# EUROPEAN PATENT APPLICATION

(11) **EP 3 904 507 A1**
(43) Date of publication of application: **03.11.2021**
(21) Application number: 20171909.3
(22) Date of filing: 28.04.2020
(51) Int. Cl.: C12N 5/0783

(54) **METHOD TO PRODUCE T CELLS AND USES THEREOF**

(71) Applicant: Ospedale San Raffaele S.r.l., 20132 Milano (IT)
(72) Inventor: Camisa, Barbara, 20132 Milano (IT); Casucci, Monica, 20132 Milano (IT); Arcangeli, Silvia, 20132 Milano (IT); Mezzanotte, Claudia, 20132 Milano (IT); Falcone, Laura, 20132 Milano (IT); Bonini, Maria Chiara, 20132 Milano (IT)
(74) Representative: Capasso, Olga

(57) **Abstract**

The present invention refers to a method to produce a T cell with advantageous properties. The invention also refers to a T cell or an engineered T cell produced by the method and its use in therapy.

## Description

### TECHNICAL FIELD

The present invention refers to a method to produce a T cell with advantageous properties. The invention also refers to a T cell or an engineered T cell produced by the method and its use in therapy.

### BACKGROUND ART

CAR T-cell therapy has considerably changed the landscape of treatment options for B-cell malignancies, leading to the recent approval of the first two CAR T-cell products for treating cancer.¹⁻⁵ However, frequent relapses in treated patients, together with inability to achieve complete remission in certain disease types,^{4,6-8} highlight the need of further potentiating this therapeutic strategy.⁹ In addition, manifestation of severe toxicities, such as cytokine release syndrome (CRS) and neurotoxicity, still needs to be efficiently counteracted without limiting functionality.^{10,11}

Extensive clinical experience has indicated that primary objective responses are strictly associated with the level of CAR T-cell expansion early after infusion, while long-term persistence is required to prevent relapses.^{3,12,13} At the same time, however, factors associated with enhanced CAR T-cell proliferation *in vivo,* such as higher peak CAR T-cell expansion, as well as larger tumor burdens, cyclophosphamide-fludarabine lympho-depletion regimens and greater CAR T-cell doses strongly correlate with the incidence and severity of CRS and neurotoxicity.^{10,14-16}

Among others, intrinsic T-cell properties and composition of the infused T-cell product have been reported to significantly shape CAR T-cell fitness.^{17,18} Indeed, T cells exist in a wide range of interconnected differentiation statuses, extensively differing in terms of proliferative capacity, self-renewal capabilities and long-term survival.^{12,17,19} In this regard, cumulating evidence in mice and humans suggests that T-cell differentiation negatively correlates with long-term antitumor activity, with early memory T cells holding the most favorable features.^{12,19} Accordingly, T cells from chronic lymphocytic leukemia patients who responded to CD19 CAR T cells were found enriched in gene expression profiles involved in early memory, or were rather the result of a single central memory T-cell (T_{CM}) clone deriving from a TET2-targeted insertional mutagenesis event^{4,20,21}.

Recently, the identification of stem memory T cells (T_{SCM}), embodying the apex of T-cell differentiation hierarchy,^{12,22,23} paved the way for the employment of this T-cell source for cancer immunotherapy. Since T_{SCM} are extremely rare in the peripheral circulation, several efforts have been dedicated to the development of robust manufacturing protocols capable of generating and expanding this cell subset *in vitro.*^{12,17,23-27} In particular, it has been reported that pre-selection of naive T cells (T_{N}) before manipulation represents a crucial step for enriching T_{SCM} and improving the transcriptional and metabolic features of the final T-cell product.^{17,27} Indeed, the presence of more differentiated T cells during T_{N} stimulation has been reported to accelerate their functional, transcriptional and metabolic differentiation, due to intercellular "quorum sensing" mechanisms.²⁸ Albeit the superior anti-tumor activity of T_{N}-derived CAR T cells has been already profiled, a thorough evaluation of their functional behavior in complex animal models is still lacking, especially as regard toxicity, which is particularly warranted due to the typical T_{N/SCM} superior expansion capability.

Therefore, there is still the need for methods to generate T cells with improved properties for therapeutic uses.

### SUMMARY OF THE INVENTION

In the present invention it was surprisingly found that the preselection of a population of mixed CD45RA⁺/CD62L⁺/CD95⁻ T cells (T naive, T_{N}) and CD45RA⁺/CD62L⁺/CD95⁺ T cells (T stem cell memory, T_{SCM}) allow the generation of T cells or engineered T cells with superior properties, making them particularly suitable for the treatment of auto-immune disease, infections and solid or hematopoietic cancer.
Capability of CAR T cells to expand and persist in patients emerged as a key factor to achieve complete responses and prevent relapses. These features are typical of early memory T cells, which can be highly enriched by exploiting optimized manufacturing protocols. Presently, however, whether pre-selecting specific memory T-cell subsets before manipulation would be really beneficial is still an open issue, especially as regard toxicity. Therefore, inventors deeply investigated the efficacy and safety profiles of T cells generated from isolated naive/stem memory T cells (T_{N/SCM}), as compared to those derived from unselected T cells (T_{BULK}). CAR T_{N/SCM} displayed a reduced *in vitro* effector signature, compared to CAR T_{BULK}. However, when challenged against tumor cells in HSPC-humanized mice, limiting doses of CAR T_{N/SCM} showed superior antitumor activity and the unique ability to protect mice from leukemia re-challenge. Improved efficacy was associated with higher expansion rates, persistence and a more favorable T-cell phenotype, characterized by early memory preservation, strong activation and poor exhaustion. Notably, at limiting doses and low tumor burdens no cases of severe CRS (sCRS) were reported. Conversely, when infusing high CAR T-cell doses in mice with high tumor burdens, detrimental CRS and multifocal brain hemorrhages were only elicited by CAR T_{BULK}, indicating that CAR T_{N/SCM} are intrinsically less toxic. These results demonstrate that CAR T_{N/SCM} can induce deeper and more durable anti-tumor responses in the absence of sCRS and neurotoxicity, significantly widening the therapeutic index of current CAR T-cell approaches.
1. T_{N/SCM}-derived CAR T cells elicit durable antitumor responses due to higher expansion rates, preserved early memory and poor exhaustion
2. T_{N/SCM}-derived CAR T cells are intrinsically less prone to cause severe cytokine release syndrome and neurotoxicity

In this work, the inventors revised the HSPC-humanized mouse model they recently developed,²⁹ which is capable of recapitulating CAR T-cell related toxicities at the pathophysiological level, to investigate the efficacy and safety profiles of CAR T cells generated from pre-selected T_{N/SCM} precursors. CAR T_{N/SCM} displayed a superior capacity to elicit recall responses upon tumor re-challenge, compared to CAR T cells generated from unselected T cells. Surprisingly, such increased potency was especially associated with absence of CRS and neurotoxicity manifestations, rendering these cells a valuable option to widen the therapeutic index of current T-cell therapies, in particular CAR T-cell therapies or TCR cell therapies.
Therefore, the invention provides a method to produce a T cell comprising the step of:
a) isolating a population of CD45RA⁺/CD62L⁺/CD95⁻ T cells and CD45RA⁺/CD62L⁺/CD95⁺ T cells from a biological sample of a subject;
b) activating said population of T cells by stimulating CD3 and CD28;
c) contacting said activated population of T cells with IL-7 and IL-15.
Preferably said produced T cell has at least one of the following properties: prevent cytokine release syndrome, prevent neurotoxicity, display a high expansion rate, preserved early memory phenotype, a poor exhausted profile and long-term persistence.
High expansion rate means that this cell population has a higher expansion rate after *in vivo* infusion, as compared to T cell products derived from unselected CD3+ T cells (T_{bulk})
Preserved early memory phenotype means that this cell population keeps longer a pool of early memory T cells, comprising either T_{SCM} and T_{CM}, after *in vivo* infusion. Conversely, T cell products derived from unselected CD3+ T cells (T_{bulk}) more rapidly differentiate into T_{EM} and T_{TE}.

A poor exhausted profile means that this cell product displays a poorly exhausted phenotype after *in vivo* infusion, characterized by co-expression of activation markers and limited enrichment of inhibitory receptors. Conversely, T cell products derived from unselected CD3+ T cells (T_{bulk}) are characterized by an exhausted phenotype, co-expressing multiple inhibitory receptors in the absence of activation markers.
Long-term persistence means that since this cell product is enriched in "younger" T cells, characterized by an improved self-renewal potential, it can persist longer after in vivo infusion.
In a preferred embodiment the population of CD45RA⁺/CD62L⁺/CD95⁻ T cells and CD45RA⁺/CD62L⁺/CD95⁺ T cells comprise about 60 to 80 % of CD45RA⁺/CD62L⁺/CD95⁻ T cells and 40% to 20% of CD45RA⁺/CD62L⁺/CD95⁺ T cells.
In a preferred embodiment said biological sample is: blood and other liquid samples of biological origin, solid tissue samples, tissue cultures of cells derived therefrom and the progeny thereof, isolated cells from biological samples as i.e. PBMCs, bone marrow, cord blood, iPSC-derived T cells.
Preferably the stimulation of CD3 and CD28 is carried out by a CD3 agonist and a CD28 agonist, preferably the stimulation is carried out by an antibody specific for CD3 and an antibody specific for CD28. Said antibodies being activating antibodies. The stimulation of CD3 and CD 28 may be performed according to any known method in the art for instance beads, matrix or cell-free matrix.
Preferably the stimulated population of T cells is contacted with IL-7 in an amount of 10-100 U/ml, preferably 25U/ml.
Preferably the stimulated population of T cells is contacted with IL-15 in an amount of 1-500 U/ml, preferably 50U/ml.
Preferably the stimulated population of T cells is contacted for 14 days.
In a preferred embodiment the method further comprises introducing in said population of T cells a nucleic acid sequence encoding an exogenous gene, thereby producing an engineered T cell.
Preferably the exogenous gene encodes an antigen-recognizing receptor, an ortho-receptor, an immunomodulatory cytokine, a chemokine receptor, a dominant negative receptor (for instance PD1 DDR as disclosed in Cherkassky L JCI 2016, PMID: 27454297), a transcription factor able to prevent exhaustion (such as c-june as disclosed in Lynn Nature 2019, PMID: 31802004), preferably the antigen is a tumor antigen, a self-antigen or a pathogen antigen.
Preferably said antigen recognizing receptor is a T cell receptor (TCR) or a chimeric antigen receptor (CAR). Preferably it is CD 19, CD28, 41 bb.

In one embodiment, an endogenous gene encoding a TCR α chain and/or an endogenous gene encoding a TCR β chain in the cell is disrupted, preferably such that the endogenous gene encoding a TCR α chain and/or the endogenous gene encoding a TCR β chain is not expressed. In one embodiment, the endogenous gene encoding a TCR α chain and/or the endogenous gene encoding a TCR β chain is disrupted by insertion of an expression cassette comprising a polynucleotide sequence encoding a TCR of the present invention. In one embodiment, one or more endogenous genes encoding an MHC in the cell is disrupted, preferably wherein the cell is a non-alloreactive universal T-cell. In one embodiment, an endogenous gene involved in persistence, expansion, activity, resistance to exhaustion/senescence/inhibitory signals, homing capacity, or other T-cell functions in the cell is disrupted, preferably wherein the endogenous gene involved in persistence, expansion, activity, resistance to exhaustion/senescence/inhibitory signals, homing capacity, or other T-cell functions is selected from the group consisting of PD1, TIM3, LAG3, 2B4, KLRG1, TGFbR, CD160 and CTLA4. In one embodiment, the endogenous gene involved in persistence, expansion, activity, resistance to exhaustion/senescence/inhibitory signals, homing capacity, or other T-cell functions is disrupted by integration of an expression cassette, wherein the expression cassette comprises a polynucleotide sequence encoding a TCR of the present invention.
Preferably said antigen recognizing receptor is exogenous.
Preferably said nucleic acid sequence is introduced by a vector.
A vector is a tool that allows or facilitates the transfer of an entity from one environment to another. In accordance with the present invention, and by way of example, some vectors used in recombinant nucleic acid techniques allow entities, such as a segment of nucleic acid (e.g. a heterologous DNA segment, such as a heterologous Cdna segment), to be transferred into a target cell. The vector may serve the purpose of maintaining the heterologous nucleic acid (DNA or RNA) within the cell, facilitating the replication of the vector comprising a segment of nucleic acid, or facilitating the expression of the protein encoded by a segment of nucleic acid. Vectors may be non-viral or viral. Examples of vectors used in recombinant nucleic acid techniques include, but are not limited to, plasmids, chromosomes, artificial chromosomes and viruses. The vector may be single stranded or double stranded. It may be linear and optionally the vector comprises one or more homology arms. The vector may also be, for example, a naked nucleic acid (e.g. DNA). In its simplest form, the vector may itself be a nucleotide of interest.

The vectors used in the invention may be, for example, plasmid or virus vectors and may include a promoter for the expression of a polynucleotide and optionally a regulator of the promoter.
Vectors comprising polynucleotides used in the invention may be introduced into cells using a variety of techniques known in the art, such as transformation, transfection and transduction. Several techniques are known in the art, for example transduction with recombinant viral vectors, such as retroviral, lentiviral, adenoviral, adeno-associated viral, baculoviral and herpes simplex viral vectors, Sleeping Beauty vectors; direct injection of nucleic acids and biolistic transformation.
Non-viral delivery systems include but are not limited to DNA transfection methods. Here, transfection includes a process using a non-viral vector to deliver a gene to a target cell. Typical transfection methods include electroporation, DNA biolistics, lipid-mediated transfection, compacted DNA-mediated transfection, liposomes, immunoliposomes, lipofectin, cationic agent-mediated transfection, cationic facial amphiphiles (CFAs) (Nature Biotechnology 1996 14; 556) and combinations thereof.
The term "transfection" is to be understood as encompassing the delivery of polynucleotides to cells by both viral and non-viral delivery.
Transposable elements are non-viral gene delivery vehicles found ubiquitously in nature. Transposon-based vectors have the capacity of stable genomic integration and long-lasting expression of transgene constructs in cells.
Preferably said nucleic acid sequence is placed at an endogenous gene locus of the T cell.
Preferably said introduction of the nucleic acid sequence disrupts or abolishes the endogenous expression of a TCR.
The invention also provides a T cell or an engineered T cell produced by the method of the invention. Preferably said produced T cell or an engineered T cell is isolated.
The invention also provides an isolated engineered CD45RA⁺/CD62L⁺/CD95⁻ T cells and CD45RA⁺/CD62L⁺/CD95⁺ T cell population comprising a nucleic acid sequence encoding an exogenous gene wherein said population reduces at least one symptom of cytokine release syndrome (CRS) or reduces at least one symptom of neurotoxicity in a subject or wherein said population has high expansion rate.
The invention also provides an isolated activated population of CD45RA⁺/CD62L⁺/CD95⁻ T cells and CD45RA⁺/CD62L⁺/CD95⁺ T cells comprise about 60 to 80 % of CD45RA⁺/CD62L⁺/CD95⁻ T cells and 40% to 20% of CD45RA⁺/CD62L⁺/CD95⁺ T cells, wherein said population reduces at least one symptom of cytokine release syndrome (CRS) or reduces at least one symptom of neurotoxicity in a subject or wherein said population has high expansion rate.
Preferably said T cell or engineered T cell or isolated engineered T cell population or a pharmaceutical composition comprising the same is for use in a therapy, preferably for use in reducing tumor burden or for use in treating and/or preventing a neoplasm or for use in lengthening survival of a subject having a neoplasm or for use in the treatment of an infection or for use in the treatment of an autoimmune disease, preferably the neoplasm is selected from the group consisting of solid or blood cancer, preferably B cell leukemia, multiple myeloma, acute lymphoblastic leukemia (ALL), chronic lymphocytic leukemia, acute myeloid leukemia (AML), non- Hodgkin's lymphoma, preferably the neoplasm is B cell leukemia, multiple myeloma, lymphoblastic leukemia (ALL), chronic lymphocytic leukemia, or non- Hodgkin's lymphoma.
Preferably said T cell or engineered T cell or isolated engineered T cell population or a pharmaceutical composition comprising the same is for use in preventing and/or reducing at least one symptom of cytokine release syndrome (CRS) or for use in reducing at least one symptom of neurotoxicity in a subject.
Preferably the at least one symptom of cytokine release syndrome is reducing the level of at least one cytokine or chemokine or other factor selected from the group consisting of: IL-1 alpha, IL-1 beta, IL-6, IL-8, IL-10, TNF-a, IFN-g, IL-5, IL-2, IL-4, G-CSF, GM-CSF, M-CSF, TGF-b, IL-12, IL-15, and IL-17, IP-10, MIP-1-alpha, MCP1, von Willebrand factor, Angiopoietin 2, SAA, protein C reactive, ferritin.
In the present invention T_{N/SCM} is a mixed population of T_{N} and T_{SCM} as defined in the below table.

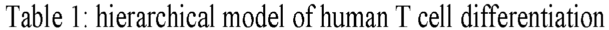

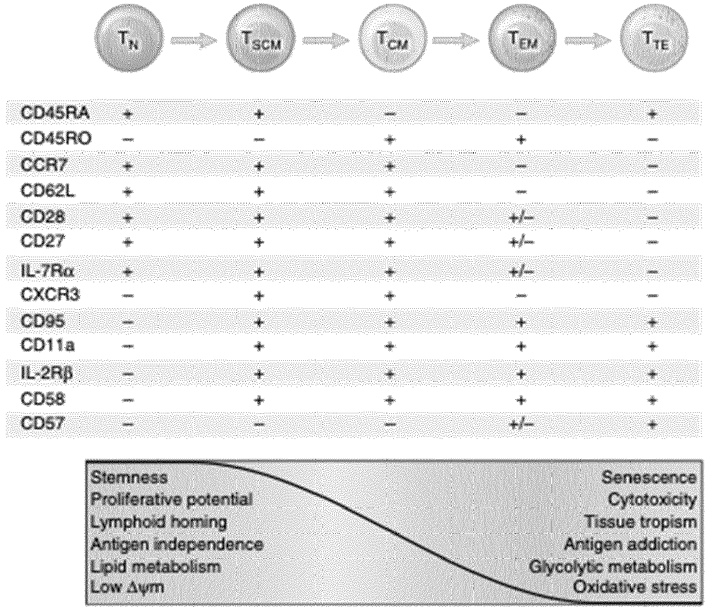

T_{N} are antigen-unexperienced T cells defined as CD3+CD45RA+CD62L+CD95-CD45RO-CCR7+ CD28+CD27+IL-7Ra+CXCR3-CD11a-IL-2Rb-CD58-CD57-. T_{N} are 38,4% +/- 12,2 (Mean +/- SD) of total CD3+ cells in healthy donors. T_{N} represent the 75,5% +/- 11,9 (Mean +/- SD) of CD3+CD45RA+CD62L+ cells (including both CD95+ and CD95- cells) in healthy donors. They are reduced in numbers in heavily pretreated cancer patients (18,8% +/- 12,9 of total CD3+ cells and 67,1% +/- 26,7 of CD3+CD45RA+CD62L+ cells in ALL patients, and 17,6% +/- 13,2 of total CD3+ cells and 62,1% +/- 17,3 of CD3+CD45RA+CD62L+ cells in patients with pancreatic cancer).
T_{SCM} are antigen-experienced T cells defined as CD3+CD45RA+CD62L+CD95+CD45RO-CCR7+CD28+CD27+IL-7Ra+CXCR3+CD11a+IL-2Rb+CD58+CD57- and endowed with stem cell-like ability to self-renew and reconstitute the entire spectrum of memory and effector T cell subset. T_{SCM} cells occupy the apex of the hierarchical system of memory T lymphocytes. T_{SCM} are 11,6% +/- 4,4 (Mean +/- SD) of total CD3+ cells in healthy donors. T_{SCM} represent the 24,5% +/- 11,9 (Mean +/- SD) of CD3+CD45RA+CD62L+ cells (including both CD95+ and CD95- cells) in healthy donors. They are reduced in numbers in heavily pretreated cancer patients (5,8% +/- 3,7 of total CD3+ cells and 32,9% +/- 26,7 of CD3+CD45RA+CD62L+ cells in ALL patients, and 7,9% +/- 3,3 of total CD3+ cells and 37,9% +/- 17,3 of CD3+CD45RA+CD62L+ cells in patients with pancreatic cancer).

T_{CM} are antigen-experienced T cells defined as CD3+CD45RA-CD62L+CD95+CD45RO+CCR7+CD28+CD27+IL-7Ra+CXCR3+CD11a+IL-2Rb+CD58+CD57-
T_{EM} are antigen-experienced T cells defined as CD3+CD45RA-CD62L-CD95+CD45RO+CCR7-CD28-CD27-IL-7Ra+/-CXCR3-CD11a+IL-2Rb+CD58+CD57+/- T_{TE} are antigen-experienced T cells defined as CD3+CD45RA+CD62L-CD95+CD45RO-CCR7-CD28-CD27-IL-7Ra-CXCR3-CD11a+IL-2Rb+CD58+CD57+
T_{BULK} are total T cells defined as CD3+. They comprise T_{N}, T_{SCM}, T_{CM}, T_{EM} and T_{TE}
CRS can present with a variety of symptoms ranging from mild, flu-like symptoms to severe life-threatening manifestations of the overshooting inflammatory response. Mild symptoms of CRS include fever, fatigue, headache, rash, arthralgia, and myalgia. More severe cases are characterized by hypotension as well as high fever and can progress to an uncontrolled systemic inflammatory response with vasopressor-requiring circulatory shock, vascular leakage, disseminated intravascular coagulation, and multi-organ system failure. Laboratory abnormalities that are common in patients with CRS include cytopenias, elevated creatinine and liver enzymes, deranged coagulation parameters, and a high CRP.
Respiratory symptoms are common in patients with CRS. Mild cases may display cough and tachypnea but can progress to acute respiratory distress syndrome (ARDS) with dyspnea, hypoxemia, and bilateral opacities on chest X-ray. ARDS may sometimes require mechanical ventilation. Of note, in patients with CRS the need for mechanical ventilation is oftentimes not due to respiratory distress but instead a consequence of the inability to protect the airway secondary to neurotoxicity. Patients with severe CRS can also develop renal failure or signs of cardiac dysfunction with reduced ejection fraction on ultrasound. In addition, patients with severe CRS frequently display vascular leakage with peripheral and pulmonary edema.
In severe cases CRS can be accompanied by clinical signs and laboratory abnormalities that resemble hemophagocytic lymphohistiocytosis (HLH) or macrophage activation syndrome (MAS). Patients with CRS-associated HLH display the typical clinical and laboratory findings of HLH/MAS such as high fevers, highly elevated ferritin levels, and hypertriglyeridemia.
Some patients develop neurotoxicity after administration of T cell-engaging therapies. The symptoms of the immune effector cell-associated neurotoxicity syndrome (ICANS) might span from mild confusion with word-finding difficulty, headaches and hallucinations to aphasia, hemiparesis, cranial nerve palsies, seizures and somnolence. In case of fatal neurotoxicity, the loss of cerebral vascular integrity manifesting as multifocal hemorrhage has also been reported (Gust Cancer Discovery 2017). In the context of CAR T cell therapy, neurotoxicity represents the second most common serious adverse event and therefore the term "CAR T cell-related encephalopathy syndrome" (CRES) has been introduced [Neelapu SS, Tummala S, Kebriaei P, Wierda W, Gutierrez C, Locke FL, et al. Chimeric antigen receptor T-cell therapy - assessment and management of toxicities. Nat Rev Clin Oncol. 2018;15:47-62. doi: 10.1038/nrclinonc.2017.148.]. There was a significant correlation between neurotoxicity and the presence and severity of CRS (Santomasso BD Cancer Discovery 2018; Gust Cancer Discovery 2017). However, distinct timing and response to treatment indicate that neurotoxicity should be excluded from the definition of CRS and treated as a separate entity. The mechanisms that lead to neurotoxicity remain unknown, but data from patients and animal models suggest there is compromise of the blood-brain barrier, associated with high levels of cytokines in the blood and cerebrospinal fluid, as well as endothelial activation.
Serum samples of patients with CAR-T associated CRS and neurotoxicity have elevated levels of IL-1β, IL-1Rα, IL-2, IL-6, IFN-γ, IL-8 (CXCL8), IL-10, IL-15, GM-CSF, G-CSF, MIP-1α/β, MCP-1 (CCL2), CXCL9, and CXCL10 (IP-10). Similarly, also markers of endothelial activation, such as the von Willebrand factor and Angiopoietin-2, are frequently elevated (Hay, Blood 2018; Gust Cancer Discovery 2017). A great effort nowadays is dedicated to the definition of early biomarkers to identify patients at risk of developing CRS and neurotoxicity. Among others, strong CRS biomarkers 36h after CAR-T infusion are a fever ≥38.9°C and elevated levels of MCP-1 in serum. Similarly, neurotoxicity biomarkers incorporated these parameters and elevated serum IL-6 levels. Many of the cytokines elevated in CRS and neurotoxicity are not produced by CAR-T cells, but by myeloid cells that are pathogenically licensed through T-cell-mediated activating mechanisms. For example, in vitro co-culture experiments and complex in vivo models have demonstrated that IL-6, MCP-1, and MIP-1 are not produced by CAR-T cells, but rather by inflammatory myeloid lineage cells (Norelli Nat Med 2018; Givridis Nat Med 2018). Key therapeutic targets to abrogate hyper-inflammation in CRS are IL-1, IL-6, and GM-CSF.
In the first place, CRS refers to the constellation of symptoms occurring after CAR T cell therapy and other immune effector cell therapies. In addition to adoptive T-cell therapies, CRS has been observed after treatment with agents differently activating T and/or other immune effector cells, such as blinatumomab, a bi-specific T cell engaging molecule consisting of 2 covalently linked single chain antibody fragments targeting CD3 on T cells and CD19 on normal and malignant B cells. CRS has also arisen with biotherapeutics intended to suppress the immune system through receptors on white blood cells. Indeed, muromonab-CD3, an anti-CD3 monoclonal antibody intended to suppress the immune system to prevent rejection of organ transplants; alemtuzumab, which is anti-CD52 and used to treat blood cancers as well as multiple sclerosis and in organ transplants; and rituximab, which is anti-CD20 and used to treat blood cancers and auto-immune disorders, all cause CRS.
In addition, severe CRS or cytokine reactions can occur in a number of infectious and non-infectious diseases including graft-versus-host disease (GVHD), coronavirus disease 2019 (COVID-19), acute respiratory distress syndrome (ARDS), sepsis, Ebola, avian influenza, smallpox, and systemic inflammatory response syndrome (SIRS).
Although, severe acute respiratory syndrome coronavirus 2 (SARS-CoV-2) is sufficiently cleared by the early acute phase anti-viral response in most individuals, some progress to a hyperinflammatory condition, often with life-threatening pulmonary involvement. This systemic hyperinflammation results in inflammatory lymphocytic and monocytic infiltration of the lung and the heart, causing ARDS and cardiac failure. Patients with fulminant COVID-19 and ARDS have classical serum biomarkers of CRS including elevated CRP, LDH, IL-6, and ferritin.
Hemophagocytic lymphohistiocytosis and Epstein-Barr virus-related hemophagocytic lymphohistiocytosis are caused by extreme elevations in cytokines and can be regarded as one form of severe cytokine release syndrome.
Classification of grading for CRS may be found in Tables of Lee et al., (Biol Blood Marrow Transplant 25 (2019) 625_638, incorporated by reference) as follows.

**Table 2: CRS consensus grading**

| **CRS Parameter** | **Grade 1** | **Grade 2** | **Grade 3** | **Grade 4** |
|---|---|---|---|---|
| **Fever*** | Temperature ≥38°C | Temperature ≥38°C | Temperature ≥38°C | Temperature ≥38°C |

| | | With | | |
|---|---|---|---|---|
| **Hypotension** | None | Not requiring vasopressors | Requiring a vasopressor with or without vasopressin | Requiring multiple vasopressors (excluding vasopressin) |

| | | And/or^{†} | | |
|---|---|---|---|---|
| **Hypoxia** | None | Requiring low-flow nasal cannula or blow-by | Requiring high-flow nasal cannula^{†}, facemask, nonrebreather mask, or Venturi mask | Requiring positive pressure (eg. CPAP, BiPAP, intubation and mechanical ventilation) |

**Table 3: ICAN grading consensus for adult**

| **Neurotoxicity Domain** | **Grade 1** | **Grade 2** | **Grade 3** | **Grade 4** |
|---|---|---|---|---|
| **ICE score*** | 7-9 | 3-6 | 0-2 | 0 (patient is unarousable and unable to perform ICE) |
| D**epressed level of consciousness^{†}** | Awakens spontaneously | Awakens to voice | Awakens only to tactile stimulus | Patient is unarousable or requires vigorous or repetitive tactile stimuli to arouse. Stupor or coma |
| **Seizure** | N/A | N/A | Any clinical seizure focal or generalized that resolves rapidly or nonconvulsive seizures on EEG that resolve with intervention | Life-threatening prolonged seizure (>5 min): or Repetitive clinical or electrical seizures without return to baseline in between |
| **Motor findings^{‡}** | N/A | N/A | N/A | Deep focal motor weakness such as hemiparesis or paraparesis |
| **Elevated ICP/ cerebral edema** | N/A | N/A | Focal/local edema on neuroimaging ^{‡} | Diffuse cerebral edema on neuroimaging: decerebrate or decorticate posturing: or cranial nerve VI palsy: or papilledema; or Cushing's triad |

**Table 4: ICAN grading consensus for children**

| **Neurotoxicity Domain** | **Grade 1** | **Grade 2** | **Grade 3** | **Grade 4** |
|---|---|---|---|---|
| **ICE score for children age ≥ 12 years*** | 7-9 | 3-6 | 0-2 | 0 (patient is unarousable and unable to perform ICE) |
| **CAPD score for children age <12 years** | 1-8 | 1-8 | ≥9 | Unable to perform CAPD |
| **Depressed level of consciousness**^{†} | Awakens spontaneously | Awakens to voice | Awakens only to tactile stimulus | Unarousable or requires vigorous or repetitive tactile stimuli to arouse: stupor or coma |
| **Seizure (any age)** | N/A | N/A | Any clinical seizure focal or generalized that resolves rapidly or nonconvulsive seizures on EEC that resolve with intervention | Life-threatening prolonged seizure (>5 min); or Repetitive clinical or electrical seizures without return to baseline in between |
| **Motor weakness (any age)** | N/A | N/A | N/A | Deep focal motor weakness, such as hemiparesis or paraparesis |
| **Elevated ICP/ cerebral edema (any age)** | N/A | N/A | Focal/local edema on neuroimaging | Decerebrate or decorticate posturing, cranial nerve VI palsy, papilledema, Cushing's triad, or signs of diffuse cerebral edema on neuroimaging |

In the present preferably the solid tumor is selected from the group consisting of: epithelial and mesenchymal malignancies, preferably adenocarcinoma of the breast, pancreas, colon-rectum, prostate, squamous cell carcinomas of the head and neck, lung, ovary, bladder cancer; soft-tissues sarcomas and osteosarcomas. More preferably the cancer is a solid cancer selected from the group consisting of colon cancer, rectal cancer, renal-cell carcinoma, liver cancer, non-small cell carcinoma of the lung, cancer of the small intestine, cancer of the esophagus, melanoma, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the endocrine system, cancer of the thyroid gland, cancer of the parathyroid gland, cancer of the adrenal gland, sarcoma of soft tissue, cancer of the urethra, cancer of the penis, solid tumors of childhood, cancer of the bladder, cancer of the kidney or ureter, carcinoma of the renal pelvis, neoplasm of the central nervous system (CNS), primary CNS lymphoma, tumor angio genesis, spinal axis tumor, brain stem glioma, pituitary adenoma, Kaposi's sarcoma, epidermoid cancer, squamous cell cancer, T-cell lymphoma, environmentally induced cancers, combinations of said cancers, and metastatic lesions of said cancers.
Preferably the haematopoietic or lymphoid tumor is selected from the group consisting of: Leukemia, Lymphomas or Myelomas, preferably the leukemia is acute lymphoblastic leukemia (ALL), acute myelogenous leukemia (AML), chronic lymphocytic leukemia (CLL), small lymphocytic lymphoma (SLL), chronic myelogenous leukemia (CML), acute monocytic leukemia (AMoL), preferably the lymphoma is Hodgkin's lymphomas, Non-Hodgkin's lymphomas.
More preferably, the cancer is a hematologic cancer chosen from one or more of chronic lymphocytic leukemia (CLL), acute leukemias, acute lymphoid leukemia (ALL), B-cell acute lymphoid leukemia (B-ALL), T-cell acute lymphoid leukemia (T-ALL), chronic myelogenous leukemia (CML), B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, marginal zone lymphoma, multiple myeloma, myelodysplasia and myelodysplastic syndrome, non-Hodgkin's lymphoma, Hodgkin's lymphoma, plasmablastic lymphoma, plasmacytoid dendritic cell neoplasm, Waldenstrom macroglobulinemia, or preleukemia.
Preferably the infected cell is selected from the group consisting of: HIV- (human immunodeficiency virus), RSV- (Respiratory Syncytial Virus), EBV- (Epstein-Barr virus), CMV- (cytomegalovirus), HBV, HCV, adenovirus-, BK polyomavirus- , coronavirus-infected cell. In particular COVID-19-infected cells.
A variety of diseases may be ameliorated by introducing the cells of the invention to a subject suitable for adoptive cell therapy. Examples of diseases including various autoimmune disorders, including but not limited to, alopecia areata, autoimmune hemolytic anemia, autoimmune hepatitis, dermatomyositis, diabetes (type 1), some forms of juvenile idiopathic arthritis, glomerulonephritis, Graves' disease, Guillain-Barre syndrome, idiopathic thrombocytopenic purpura, myasthenia gravis, some forms of myocarditis, multiple sclerosis, pemphigus/pemphigoid, pernicious anemia, polyarteritis nodosa, polymyositis, primary biliary cirrhosis, psoriasis, rheumatoid arthritis, scleroderma/systemic sclerosis, Sjogren's syndrome, systemic lupus, erythematosus, some forms of thyroiditis, some forms of uveitis, vitiligo, granulomatosis with poly angiitis (Wegener's); hematological malignancies, including but not limited to, acute and chronic leukemias, lymphomas, multiple myeloma and myelodysplastic syndromes; solid tumors, including but not limited to, tumor of the brain, prostate, breast, lung, colon, uterus, skin, liver, bone, pancreas, ovary, testes, bladder, kidney, head, neck, stomach, cervix, rectum, larynx, or esophagus; and infections, including but not limited to, HIV- (human immunodeficiency virus), RSV- (Respiratory Syncytial Virus), EBV- (Epstein-Barr virus), CMV- (cytomegalovirus), HBV, HCV, adenovirus- and BK polyomavirus- associated disorders.

### CAR-T cell therapy

### Chimeric Antigen Receptors

"Chimeric antigen receptor" or "CAR" or "CARs" refers to engineered receptors which can confer an antigen specificity onto cells (for example T cells). CARs are also known as artificial T-cell receptors, chimeric T-cell receptors or chimeric immunoreceptors. Preferably the CARs of the invention comprise an antigen-specific targeting region, an extracellular domain, a transmembrane domain, optionally one or more co-stimulatory domains, and an intracellular signaling domain.

### Antigen-specific targeting domain

The antigen-specific targeting domain provides the CAR with the ability to bind to the target antigen of interest. The antigen-specific targeting domain preferably targets an antigen of clinical interest against which it would be desirable to trigger an effector immune response that results in tumor killing.

The antigen-specific targeting domain may be any protein or peptide that possesses the ability to specifically recognize and bind to a biological molecule (e.g., a cell surface receptor or tumor protein, or a component thereof). The antigen-specific targeting domain includes any naturally occurring, synthetic, semi-synthetic, or recombinantly produced binding partner for a biological molecule of interest.

Illustrative antigen-specific targeting domains include antibodies or antibody fragments or derivatives, extracellular domains of receptors, ligands for cell surface molecules/receptors, or receptor binding domains thereof, and tumor binding proteins.

In a preferred embodiment, the antigen-specific targeting domain is, or is derived from, an antibody. An antibody-derived targeting domain can be a fragment of an antibody or a genetically engineered product of one or more fragments of the antibody, which fragment is involved in binding with the antigen. Examples include a variable region (Fv), a complementarity determining region (CDR), a Fab, a single chain antibody (scFv), a heavy chain variable region (VH), a light chain variable region (VL) and a camelid antibody (VHH).

In a preferred embodiment, the binding domain is a single chain antibody (scFv). The scFv may be murine, human or humanized scFv.

"Complementarity determining region" or "CDR" with regard to an antibody or antigen-binding fragment thereof refers to a highly variable loop in the variable region of the heavy chain or the light chain of an antibody. CDRs can interact with the antigen conformation and largely determine binding to the antigen (although some framework regions are known to be involved in binding). The heavy chain variable region and the light chain variable region each contain 3 CDRs.

"Heavy chain variable region" or "VH" refers to the fragment of the heavy chain of an antibody that contains three CDRs interposed between flanking stretches known as framework regions, which are more highly conserved than the CDRs and form a scaffold to support the CDRs.

"Light chain variable region" or "VL" refers to the fragment of the light chain of an antibody that contains three CDRs interposed between framework regions.

"Fv" refers to the smallest fragment of an antibody to bear the complete antigen binding site. An Fv fragment consists of the variable region of a single light chain bound to the variable region of a single heavy chain.

"Single-chain Fv antibody" or "scFv" refers to an engineered antibody consisting of a light chain variable region and a heavy chain variable region connected to one another directly or via a peptide linker sequence.

Antibodies that specifically bind a tumor cell surface molecule can be prepared using methods well known in the art. Such methods include phage display, methods to generate human or humanized antibodies, or methods using a transgenic animal or plant engineered to produce human antibodies. Phage display libraries of partially or fully synthetic antibodies are available and can be screened for an antibody or fragment thereof that can bind to the target molecule. Phage display libraries of human antibodies are also available. Once identified, the amino acid sequence or polynucleotide sequence coding for the antibody can be isolated and/or determined.

Examples of antigens which may be targeted by the CAR of the invention include but are not limited to antigens expressed on cancer cells and antigens expressed on cells associated with various hematologic diseases, autoimmune diseases, inflammatory diseases and infectious diseases.

With respect to targeting domains that target cancer antigens, the selection of the targeting domain will depend on the type of cancer to be treated, and may target tumor antigens. A tumor sample from a subject may be characterized for the presence of certain biomarkers or cell surface markers. For example, breast cancer cells from a subject may be positive or negative for each of Her2Neu, Estrogen receptor, and/or the Progesterone receptor. A tumor antigen or cell surface molecule is selected that is found on the individual subject's tumor cells. Preferably the antigen-specific targeting domain targets a cell surface molecule that is found on tumor cells and is not substantially found on normal tissues, or restricted in its expression to non-vital normal tissues.

Further antigens specific for cancer which may be targeted by a CAR include but are not limited to any one or more of mesothelin, EGFRvIII, TSHR, CD19, CD123, CD22, CD30, CD171, CS-1, CLL-1, CD33, GD2, GD3, BCMA, Tn Ag, prostate specific membrane antigen (PSMA), ROR1, FLT3, FAP, TAG72, CD38, CD44v6, CEA, EPCAM, B7H3, KIT, IL-13Ra2, interleukin-11 receptor a (IL-1 IRa), PSCA, PRSS21, VEGFR2, LewisY, CD24, platelet-derived growth factor receptor- beta (PDGFR-beta), SSEA-4, CD20, Folate receptor alpha (FRa), ERBB2 (Her2/neu), MUC1, epidermal growth factor receptor (EGFR), NCAM, Prostase, PAP, ELF2M, Ephrin B2, IGF-I receptor, CAIX, LMP2, gplOO, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, GPRC5D, CXORF61, CD97, CD 179a, ALK, Polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6,E7, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B 1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, and IGLL1,

Antigens specific for inflammatory diseases which may be targeted by the CAR of the invention include but are not limited to any one or more of AOC3 (VAP-1), CAM-3001, CCL11 (eotaxin-1), CD125, CD147 (basigin), CD154 (CD40L), CD2, CD20, CD23 (IgE receptor), CD25 (a chain of IL-2 receptor), CD3, CD4, CD5, IFN-α, IFN-γ, IgE, IgE Fc region, IL-1, IL-12, IL-23, IL-13, IL-17, IL-17A, IL-22, IL-4, IL-5, IL-5, IL-6, IL-6 receptor, integrin α4, integrin α4β7, Lama glama, LFA-1 (CD11a), MEDI-528, myostatin, OX-40, rhuMAb β7, scleroscin, SOST, TGF β1, TNF-a or VEGF-A.

Antigens specific for neuronal disorders which may be targeted by the CAR of the invention include but are not limited to any one or more of beta amyloid or MABT5102A.

Antigens specific for diabetes which may be targeted by the CAR of the invention include but are not limited to any one or more of L-1β or CD3. Other antigens specific for diabetes or other metabolic disorders will be apparent to those of skill in the art.

Antigens specific for cardiovascular diseases which may be targeted by the CARs of the invention include but are not limited to any one or more of C5, cardiac myosin, CD41 (integrin alpha-IIb), fibrin II, beta chain, ITGB2 (CD18) and sphingosine-1-phosphate.

Preferably, the antigen-specific binding domain specifically binds to a tumor antigen. In a specific embodiment, the polynucleotide codes for a single chain Fv that specifically binds CD44v6 or CEA.

### Co-stimulatory domain

The CAR also comprises one or more co-stimulatory domains. This domain may enhance cell proliferation, cell survival and development of memory cells.

Each co-stimulatory domain comprises the co-stimulatory domain of any one or more of, for example, a MHC class I molecule, a TNF receptor protein, an Immunoglobulin-like protein, a cytokine receptor, an integrin, a signaling lymphocytic activation molecule (SLAM protein), an activating NK cell receptor, BTLA, a Toll ligand receptor, OX40, CD2, CD7, CD27, CD28, CD30, CD40, CDS, ICAM-1, LFA-1 (CD1 1a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD 19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD1 1d, ITGAE, CD103, ITGAL, CD1 1a, LFA-1, ITGAM, CD1 1b, ITGAX, CD1 1c, ITGB 1, CD29, ITGB2, CD 18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD 19a, and a ligand that specifically binds with CD83.. Additional co-stimulatory domains will be apparent to those of skill in the art.

### Intracellular signaling domain

The CAR also comprises an intracellular signaling domain. This domain may be cytoplasmic and may transduce the effector function signal and direct the cell to perform its specialized function. Examples of intracellular signaling domains include, but are not limited to, ζ chain of the T-cell receptor or any of its homologs (e.g., η chain, FcεR1γ and β chains, MB1 (Igα) chain, B29 (Igβ) chain, etc.), CD3 polypeptides (Δ, δ and ε), syk family tyrosine kinases (Syk, ZAP 70, etc.), src family tyrosine kinases (Lck, Fyn, Lyn, etc.) and other molecules involved in T-cell transduction, such as CD2, CD5 and CD28. The intracellular signaling domain may be human CD3 zeta chain, FcyRIII, FcsRI, cytoplasmic tails of Fc receptors, immunoreceptor tyrosine-based activation motif (ITAM) bearing cytoplasmic receptors or combinations thereof.

Preferable, the intracellular signaling domain comprises the intracellular signaling domain of human CD3 zeta chain.

### Transmembrane domain

The CAR also comprises a transmembrane domain. The transmembrane domain may comprise the transmembrane sequence from any protein which has a transmembrane domain, including any of the type I, type II or type III transmembrane proteins. The transmembrane domain of the CAR of the invention may also comprise an artificial hydrophobic sequence. The transmembrane domains of the CARs of the invention may be selected so as not to dimerize. Additional transmembrane domains will be apparent to those of skill in the art. Examples of transmembrane (TM) regions used in CAR constructs are: 1) The CD28 TM region (Pule et al, Mol Ther, 2005, Nov;12(5):933-41; Brentjens et al, CCR, 2007, Sep 15;13(18 Pt 1):5426-35; Casucci et al, Blood, 2013, Nov 14;122(20):3461-72.); 2) The OX40 TM region (Pule et al, Mol Ther, 2005, Nov;12(5):933-41); 3) The 41BB TM region (Brentjens et al, CCR, 2007, Sep 15;13(18 Pt 1):5426-35); 4) The CD3 zeta TM region (Pule et al, Mol Ther, 2005, Nov;12(5):933-41; Savoldo B, Blood, 2009, Jun 18;113(25):6392-402.); 5) The CD8a TM region (Maher et al, Nat Biotechnol, 2002, Jan;20(1):70-5.; Imai C, Leukemia, 2004, Apr;18(4):676-84; Brentjens et al, CCR, 2007, Sep 15;13(18 Pt 1):5426-35; Milone et al, Mol Ther, 2009, Aug;17(8): 1453-64.).

A T cell receptor (TCR) is a molecule which can be found on the surface of T-cells that is responsible for recognizing antigens bound to MHC molecules. The naturally-occurring TCR heterodimer consists of an alpha (α) and beta (β) chain in around 95% of T-cells, whereas around 5% of T-cells have TCRs consisting of gamma (γ) and delta (δ) chains.
Engagement of a TCR with antigen and MHC results in activation of the T lymphocyte on which the TCR is expressed through a series of biochemical events mediated by associated enzymes, co-receptors, and specialized accessory molecules.
Each chain of a natural TCR is a member of the immunoglobulin superfamily and possesses one N-terminal immunoglobulin OM-variable (V) domain, one Ig-constant (C) domain, a transmembrane/cell membrane-spanning region, and a short cytoplasmic tail at the C-terminal end.

The variable domain of both the TCR α chain and β chain have three hypervariable or complementarity determining regions (CDRs). A TCR α chain or β chain, for example, comprises a CDR1, a CDR2, and a CDR3 in amino to carboxy terminal order. In general, CDR3 is the main CDR responsible for recognizing processed antigen, although CDR1 of the alpha chain has also been shown to interact with the N-terminal part of the antigenic peptide, whereas CDR1 of the beta chain interacts with the C-terminal part of the peptide. CDR2 is thought to recognize the MHC molecule.
A constant domain of a TCR may consist of short connecting sequences in which a cysteine residue forms a disulfide bond, making a link between the two chains.
An α chain of a TCR of the present invention may have a constant domain encoded by a TRAC gene.
A β chain of a TCR of the present invention may have a constant domain encoded by a TRBC1 or a TRBC2 gene.
The present invention will be described by means of non-limiting examples in reference to the following figures.

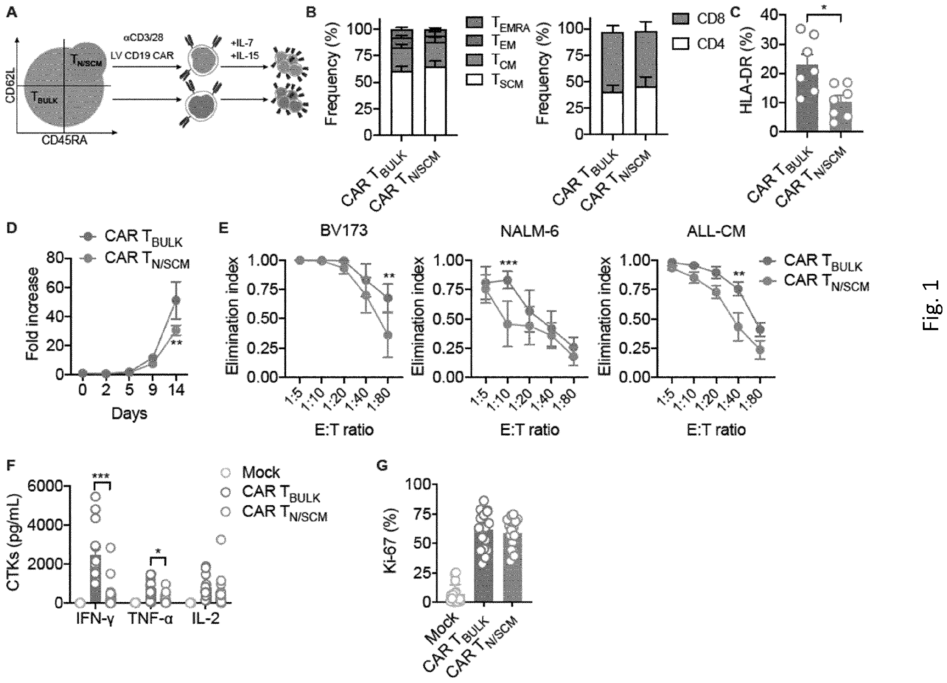

**Figure 1****. CAR T_{N/SCM} display an indolent effector signature *in vitro.* A)** Schematical representation of CAR T-cell differentiation experimental layout. Briefly, double positive CD62L+/CD45RA+ cells were FACS-sorted and bulk unselected T cells were employed as controls. T_{N/SCM} and T_{BULK} were activated with TransAct, transduced with a LV encoding for a CD19.28z CAR and cultured with IL-7/IL-15. **B**) Memory phenotype, CD4/CD8 ratio, **C**) HLA-DR expression at the end of manufacturing (n=7). **D**) Fold expansion at different time points during culture (n=4). **E**) Killing activity expressed as Elimination Index (see Methods) and measured by co-culturing CAR T cells with CD19+ targets for 4 days at different Effector to Target (E:T) ratios (n=7 for CAR T_{BULK}, n=6 for CAR T_{N/SCM} against Lucia+/NGFR+/NALM-6 and BV173 cell lines; n=6 for CAR T_{BULK}, n=6 for CAR T_{N/SCM} against ALL-CM cell line). **F**) Cytokine production after 24h co-culture of CAR T cells with CD19+ targets at the 1:10 E:T ratio (pulled data from n=5 donors challenged against Lucia+/NGFR+/NALM-6, BV173 and ALL-CM CD19+ target cell lines). **G**) T-cell proliferation after a 4-day co-culture with CD19+ targets, measured by intracellular staining of Ki-67 (pulled data from n=6 donors challenged against Lucia+/NGFR+/NALM-6, BV173 and ALL-CM CD19+ target cell lines). Data are represented as the result of mean ±SEM or mean ±SEM together with overlapping scattered values. Results of paired t-test and two-way ANOVA are reported when statistically significant (*p<0.05, **p<0.01, ***p<0.001).

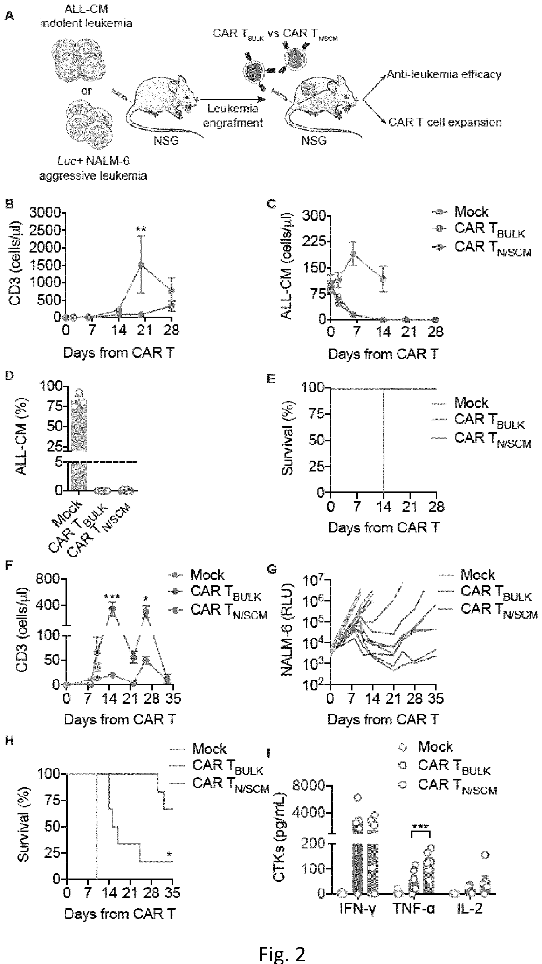

**Figure 2****. CAR T_{N/SCM} underperform CAR T_{BULK} in NSG mice. A)** Schematic representation of xenograft mouse models. NSG mice were infused with Lucia+/NGFR+/NALM-6 leukemia or ALL-CM leukemia and treated with low doses of CAR T_{N/SCM} (n=6 NALM6; n=8 ALL-CM), CAR T_{BULK} (n=6 NALM6; n=8 ALL-CM) or Mock control (n=6 NALM6; n=3 ALL-CM). **B**) T-cell expansion in the peripheral blood of ALL CM-bearing mice measured at different time points after treatment. C) Peripheral blood cell count of CD10+/CD19+ ALL-CM cells in treated mice. **D**) Analysis of ALL-CM leukemic cell infiltration in the bone marrow of treated mice at sacrifice. Dotted line identifies the threshold defining deep remission (BM ALL-CM < 5%)²⁹. **E**) Survival curves of ALL-CM bearing mice treated with different T-cell products. Data are represented as Kaplan-Meyer survival plots and the results of a Mantel-Cox two-sided log-rank test was reported when statistically significant. **F**) T-cell expansion in the peripheral blood of NALM-6 bearing mice measured at different time points after treatment. **G**) NALM-6-derived bioluminescence signal measured at different time points after treatment and expressed as Relative Light Units (RLU). Each line represents a single treated mouse. **H**) Survival curves of NALM-6 bearing mice treated with different T-cell products. Data are represented as Kaplan-Meyer survival plots and the results of a Mantel-Cox two-sided log-rank test are reported when statistically significant. **I**) Pro-inflammatory cytokine levels in NALM-6 bearing mice measured at day 13 after treatment. Data are represented as the result of mean ±SEM or mean ±SEM together with overlapping scattered values. Results of unpaired t-test and two-way ANOVA are reported when statistically significant (*p<0.05, **p<0.01, ***p<0.001).

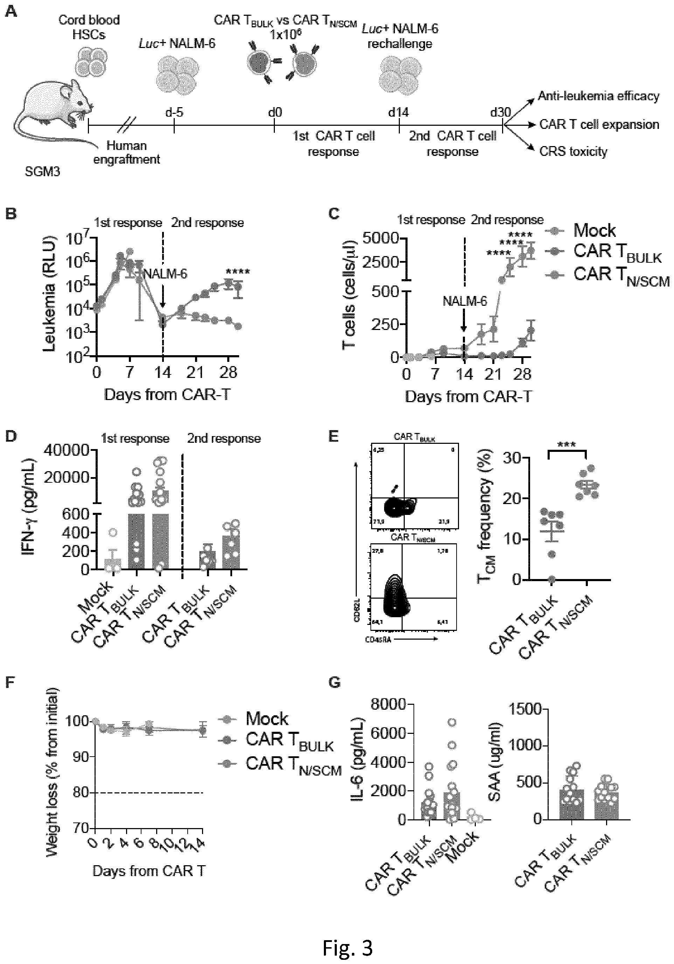

**Figure 3****. CAR T_{N/SCM} display superior anti-tumor activity and expansion in HuSGM3 mice. A)** Schematic representation of the HSCP-humanized mouse model. SGM3 mice were infused with HSPCs and, after hematopoietic reconstitution, injected with Lucia+/NGFR+/NALM-6 leukemia and treated with low doses of CAR T_{N/SCM} (n=17), CAR T_{BULK} (n=17) or Mock control (n=7). **B**) NALM-6-derived bioluminescence signal measured at different time points after treatment and expressed as Relative Light Units (RLU). **C**) T-cell expansion in the peripheral blood of NALM-6 bearing mice measured at different time points after treatment. **D**) IFN-γ serum levels measured at day 4 after treatment and day 5 after NALM-6 re-challenge. **E**) T-cell memory phenotype of CAR T_{BULK} and CAR T_{N/SCM} at day 14 after treatment. Left panel: dot plot of two representative mice. Right panel: frequency of the central memory T-cell subset (analysis performed for n=7 mice/group). **F, G**) Evaluation of signs and symptoms typical of CRS development in HuSGM3 leukemia bearing mice after treatment, represented by weight loss, IL-6 and murine SAA serum levels. Data are represented as the result of mean ±SEM or mean ±SEM together with overlapping scattered values. Results of unpaired t-test and two-way ANOVA are reported when statistically significant (***p<0.001; ****p<0.0001).

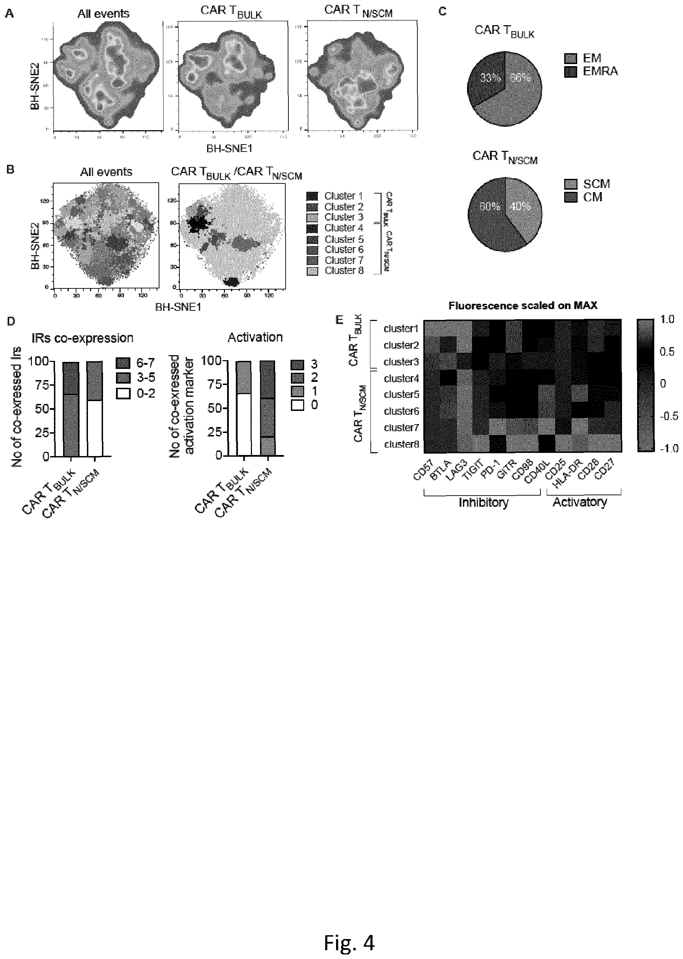

**Figure 4****. CAR T_{N/SCM} retain an enhanced *in vivo* fitness after leukemia encounter**. SGM3 mice were infused with HSPCs and, after hematopoietic reconstitution, injected with Lucia+/NGFR+/NALM-6 leukemia and treated with low doses of CAR T_{N/SCM} (n=3) or CAR T_{BULK} (n=5). **A**) A median of ∼74000 CD3+ lymphocytes deriving from peripheral blood of both CAR T_{N/SCM} and CAR T_{BULK} treated mice at day 14 after leukemia infusion were inquired by BH-SNE and K-means algorithms. Data were plotted according to BH-SNE1 and BH-SNE2 specifically calculated variables and the events were split in two density plots according to the CAR T-cell population they belong to. **B**) After application of Flow-SOM algorithm to both BH-SNE1 and BH-SNE2 variables, the resulting identified clusters were attributed to CAR T_{N/SCM} and CAR T_{BULK} groups. **C**, **D**) CAR T_{N/SCM} and CAR T_{BULK} specific clusters were described in terms of T-cell memory subset composition, together with expression of inhibitory and activation receptors. **E**) Heatmap visualization of both inhibitory and activation receptors expressed by CAR T_{N/SCM} and CAR T_{BULK} specific meta-clusters, in which MFI levels were normalized on the basis of the maximum expressed value of each analyzed parameter in the whole analyzed sample.

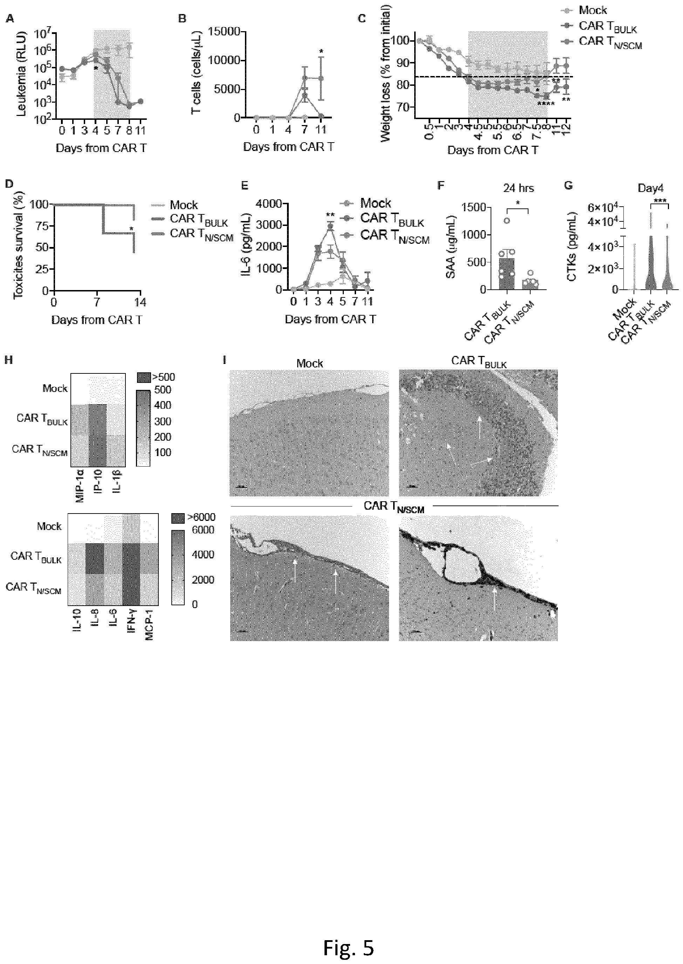

**Figure 5****. CAR T_{N/SCM} display an intrinsically reduced toxic profile.** SGM3 mice were infused with 10⁵ HSPCs and, after hematopoietic reconstitution, injected with Lucia+/NGFR+/NALM-6 leukemia. When a high tumor burden was reached, mice were treated with high doses of CAR T_{N/SCM} (n=6), CAR T_{BULK} (n=6) or Mock control (n=3). **A**) NALM-6-derived bioluminescence signal measured at different time points after treatment and expressed as Relative Light Units (RLU). **B**) T-cell expansion in the peripheral blood of NALM-6 bearing mice. C) Weigh loss evaluation at different time points after treatment. **D**) sCRS- and neurotoxicity-related survival curves of NALM-6 bearing HuSGM3 mice treated with different T-cell products. Data are represented as Kaplan-Meyer survival plots and the results of a Mantel-Cox two-sided log-rank test are reported when statistically significant. **E**) Kinetic of IL-6 serum levels at different time points after treatment. **F**) Murine SAA serum levels 24 hours after treatment. **G**) Peak serum cytokine levels at day 4 after treatment. Data are represented as the result of mean ±SEM or mean ±SEM together with overlapping scattered values. Results of unpaired t-test and two-way ANOVA are reported when statistically significant (*p<0.05, **p<0.01, ***p<0.001; ****p<0.0001). **H**) Heatmap visualization of peak serum cytokine levels at day 4 after treatment. Data are represented as the result of mean ±SEM and values are scaled according to a colored-graded range depending on relative minimum and maximum levels, respectively. **I**) Hematoxylin and eosin stained sections of brains belonging to representative Mock control (upper left), CAR T_{BULK} (upper right) and CAR T_{N/SCM} (lower left) treated mice (20x magnification; bar: 50 micron; white arrows identify foci of hemorrhages within the parenchyma). Lower right panel: Immunohistochemical characterization of CD3+ cells in brain sections of CAR T_{N/SCM} treated mice (20x magnification bar: 50 micron).

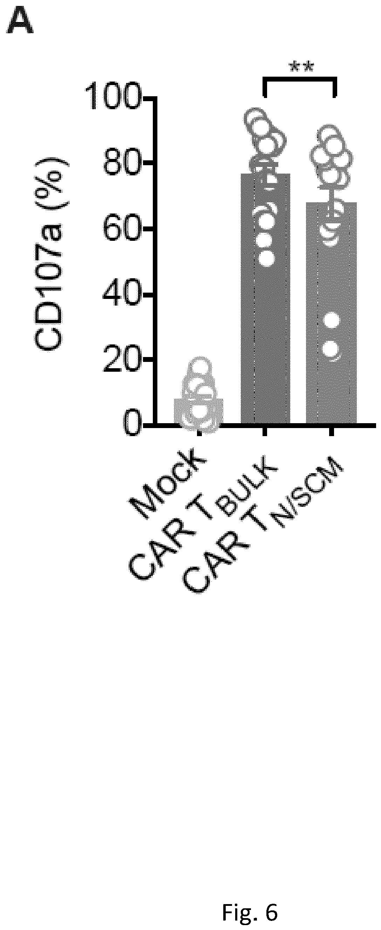

**Figure 6****. Degranulation activity of CAR T_{N/SCM} and CAR T_{BULK} *in vitro.* A)** De-granulation assays performed by co-culturing CAR T_{N/SCM}, CAR T_{BULK} and Mock control with CD19+ targets for 24 hours (pulled data deriving from n=7 donors challenged against Lucia+/NGFR+/NALM-6, BV173 and ALL-CM CD19+ target cell lines). Data are represented as the result of mean ±SEM together with overlapping scattered values. Results of unpaired t-test are reported when statistically significant (**p<0.01).

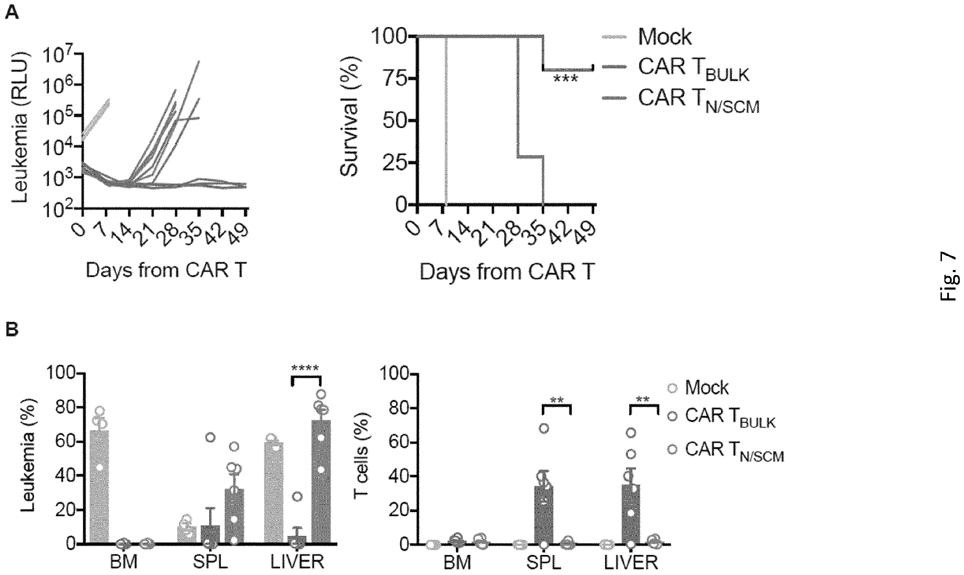

**Figure 7****. CAR T_{N/SCM} underperform CAR T_{BULK} in NSG mice independently of the CAR T-cell dose employed.** NSG mice were infused with Lucia+/LNGFR+/NALM-6 cells and treated with 3x10⁶ CAR T_{N/SCM}, CAR T_{BULK} or control untransduced T cells (Mock). A) NALM-6-derived bioluminescence signal measured at different time points after treatment and expressed as Relative Light Units (RLU). Each line represents a single treated mouse. Right panel, survival curves of NALM-6-bearing mice treated with different T-cell products (n=6 mice/group, n=4 Mock control). Data are represented as Kaplan-Meyer survival plots for mice and the results of a Mantel-Cox two-sided log-rank test are reported when statistically significant (***p<0.001). **B**) Analysis of NALM-6 cell (left panel) and T-cell (right panel) infiltration in the bone marrow, spleen and liver of treated mice (n=6 mice/group, n=4 Mock control). Data are represented as the result of mean ±SEM together with overlapping scattered values. Results of unpaired t-test analysis are reported when statistically significant (**p<0.01, ****p<0.0001).

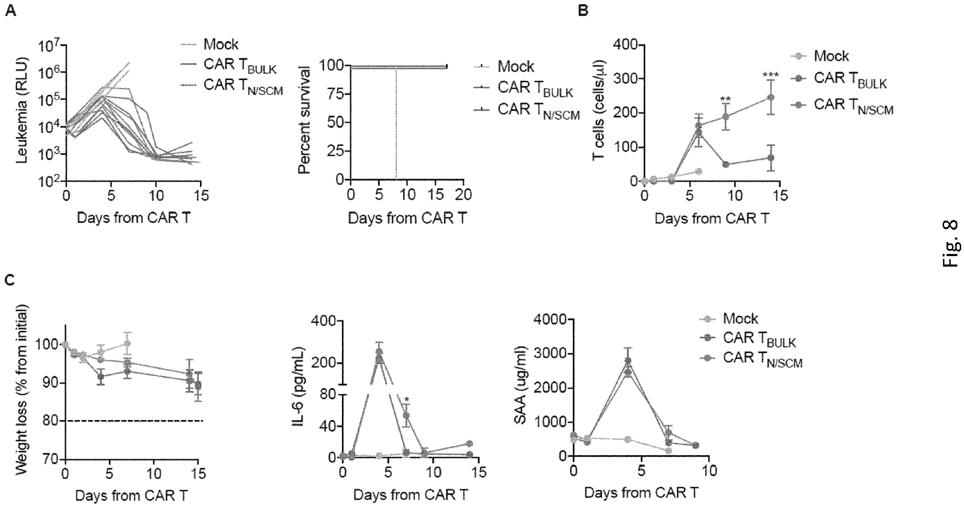

**Figure 8****. CAR T_{N/SCM} display an enhanced expansion in HuSGM3 mice without causing detrimental side effects. A)** Left panel: bioluminescence detection of Lucia+/NGFR+/NALM-6 systemic growth in HuSGM3 mice after treatment with 3x10^6 CAR T_{N/SCM} or CAR T_{BULK} and Mock control, respectively. Each line represents a single treated mouse. Right panel: survival curve of Lucia+/NGFR+/NALM-6 leukemia bearing mice treated with CAR T_{N/SCM}, CAR T_{BULK} and Mock control. Data are represented as Kaplan-Meyer survival plots for mice and result of a Mantel-Cox two-sided log-rank test are reported when statistically significant. (n=5 mice/group n=3 Mock control). **B**) CAR T_{N/SCM}, CAR T_{BULK} and Mock control expansion kinetic in the peripheral blood of Lucia+/NGFR+/NALM-6 HuSGM3 bearing mice **C**) Evaluation of signs and symptoms typical of CRS development in HuSGM3 leukemia bearing mice after T-cell infusion, represented by weight loss, IL-6 and murine SAA serum elevation levels, respectively (n=5 mice/group, n=3 Mock control). Data are represented as the result of mean ±SEM. Results of two-way ANOVA statistical analysis are reported when statistically significant (**p<0.01, ***p<0.001).

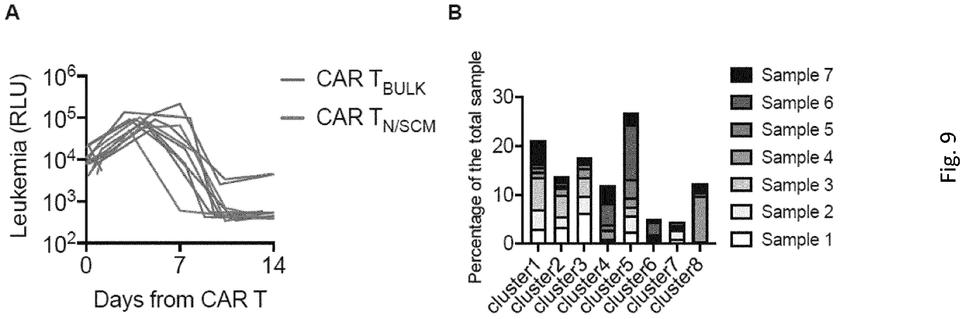

**Figure 9****. CAR T_{N/SCM} and CAR T_{BULK} are effective *in vivo* and equally represented in the meta-cluster analysis. A)** Bioluminescence detection of Lucia+/NGFR+/NALM-6 systemic growth in HuSGM3 mice after treatment with 1x10^6 CAR T_{N/SCM} or CAR T_{BULK} and Mock control, respectively. **B**) Distribution of each sample in the relevant clusters after BH-SNE analysis.

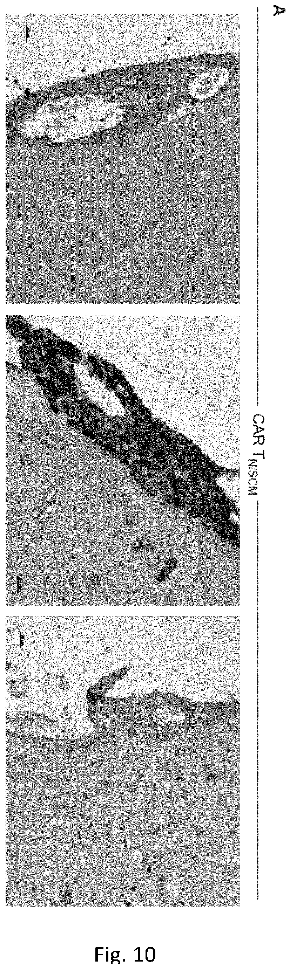

**Figure 10****. CD3+ cells infiltrate the meninges of CAR T_{N/SCM} treated mice in the absence of CD19+ cells A)** CAR T_{N/SCM} brain sections were stained with hematoxylin and eosin (left panel) and analyzed by immunohistochemistry after counterstaining with anti-hCD3 (middle panel) or anti-hCD19 monoclonal antibodies and peroxidase-conjugated second-step reagent (right panel, 40x magnification).

### DETAILED DESCRIPTION OF THE INVENTION

### Materials and methods

### Transduction and Culture Conditions

Buffy coats from healthy donors were obtained after written informed consent and IRB approval. CD45RA+/CD62L+ Naive/Stem Cell Memory T cells (T_{N/SCM}) were FACS-sorted. Unselected T cells and T_{N/SCM} were stimulated through MACS-GMP T Cell TransAct (Miltenyi), transduced with a bidirectional lentiviral vector encoding for a CD19.CAR.28z and the LNGFR marker gene. Cells were kept in culture in TexMacs medium (Miltenyi), supplemented with low-doses IL-7/IL-15 (Miltenyi) for 15 days. CAR+ cells were enriched by sorting through magnetic labelling of the LNGFR marker gene. Phenotypic and functional analysis of each CAR T-cell product were performed at the end of manufacturing.

### In Vitro Functional Assays

CAR T_{BULK} or CAR T_{N/SCM} cells were co-cultured with CD19+ leukemic cell lines (Lucia+/NGFR+/NALM-6; ALL-CM; BV-173) at different E:T ratios. Untransduced T cells were used as control (Mock). After 24h hours, supernatants were collected and analyzed with the LEGENDplex bead-based cytokine immunoassay (Biolegend). After 4 days, residual cells in culture were analyzed by FACS using Flow-Count Fluorospheres (BeckmanCoulter). The elimination index was calculated as follows: 1 - (number of residual target cells in presence of target antigen-specific CAR T cells/number of residual target cells in presence of CTRL CAR T cells). For de-granulation assays, CAR T_{BULK} or CAR T_{N/SCM} cells were labeled with FITC-anti-CD107a immediately after co-culture with different CD19+ cell lines at the 1:3 E:T ratio. After 24 hours, cells were collected and analyzed by FACS. For proliferation assays, CAR T_{BULK} or CAR T_{N/SCM} cells were co-cultured with CD19+ targets at the E:T ratio of 1:1. After 4 days, cells were stained for intracellular Ki-67 and analyzed by FACS.

### In vivo experiments

All mouse experiments were approved by the Institutional Animal Care and Use Committee (IACUC) of San Raffaele University Hospital and Scientific Institute and by the Italian Governmental Institute of Health (Rome, Italy).

Six to 8-week-old NOD.Cg-Prkdcscid IL-2rgtm1Wj1/SzJ (NSG) mice were obtained from Jackson Laboratory. In the indolent tumor model, NSG mice were injected i.v. with 8x10⁶ ALL-CM cells and, upon tumor engraftment, treated i.v. with 2x10⁶ CAR T_{BULK}, CAR T_{N/SCM} or Mock T cells. In the aggressive tumor setting, NSG mice were injected i.v. with 0.5x10⁶ Lucia+/NGFR+/NALM-6 cells and after 5 days treated i.v. with 1x10⁶ or 3x10⁶ CAR T_{BULK}, CAR T_{N/SCM} or Mock T cells. Lucia+/NGFR+/NALM-6 cells were monitored by bioluminescence detection, using the QUANTI-Luc detection reagent (InvivoGen), while ALL-CM cells and CAR T cells were monitored by FACS using Flow-Count Fluorospheres (BeckmanCoulter).³⁰

NSGTgCMV-IL3, CSF2, KITLG1Eav/MloySzJ (SGM3) mice were sub-lethally irradiated and infused i.v. with 1×10⁵ human cord blood CD34+ cells (Lonza). Upon reconstitution, HuSGM3 mice were infused i.v. with 0.5x10⁶ Lucia+/NGFR+/NALM-6 cells and 5 or 7 days later, in the low and high tumor burden setting respectively, treated i.v. with 1x10⁶ or 1x10⁷ CD19.CAR T_{BULK}, CD19.CAR T_{N/SCM} or control Mock T cells. Mice were sacrificed when Relative Bioluminescent Units exceeded the threshold of 1.5×10⁶ or when manifesting clinical signs of suffering. For evaluating CRS development, weight loss was daily monitored and the concentration of serum human cytokines (LegendPLEX, Biolegend) and mouse SAA (ELISA kit abcam) were weekly assessed according to the manufacturer instructions. Severe CRS mortality was defined as death preceded by: >20% body weight loss without recovery within 8 days after CAR T-cell infusion.

### BH-SNE analysis

BH-SNE (Barnes-Hut Stochastic Neighborhood Embedding) was applied on concatenate down sampled CD3+ events (7400 events/sample) collected from the peripheral blood of HuSGM3-NALM-6 bearing mice treated with CAR T cells, 14 days after infusion. BH-SNE algorithm analysis settings were perplexity=30000 and theta=0.5. Flow-SOM algorithm was then calculated for the cytometry variables of interest and clustered data in 50 different groups. Clusters were first studied in their composition by means of raw percentages and, when attributed to one experimental group, the mean fluorescence for the variables of interest was calculated and normalized according to the mean fluorescence of the total experimental dataset.

### Histopathological analysis

Brains from HuSGM3 mice were collected at necropsy, fixed in buffered 4% formalin, embedded in paraffin, cut and stained in GLP SR-TIGET Pathology laboratory following Good Laboratory Practices principles. Haematoxylin and eosin stained 3-µm paraffin sections were blindly and independently examined for histopathological analysis by two pathologists. Selected slides were stained with rabbit monoclonal anti-CD3 (2GV6), employing automated BenchMark Ultra Ventana in the Pathology Unit (accredited ISO 9001:2008, certification n. IT-25960). Photomicrographs were taken using the AxioCam HRc (Zeiss) with the AxioVision System SE64 (Zeiss).

### Statistical Analysis

Statistical analyses were performed with Prism Software 8.0 (GraphPad). Data are shown as Mean ± SEM with at least n=3 replicates. Datasets were analyzed with paired or unpaired Student's t-test, two-way ANOVA or the Log-rank Mantel-Cox tests depending on the experimental design. Differences with a P value <0.05 were considered as statistically significant.

### Cell lines

Leukemic cell lines NALM-6 and BV173 were purchased from the American Type Culture Collection (ATCC) and cultured in RPMI 1640 (BioWhittaker), supplemeted with 10% FBS (Lonza), 100 IU/ml penicillin/streptomycin and glutamine. ALL-CM cell line was kindly provided by Fred Falkenburg, Leiden University Medical Center and kept in culture in X-VIVO (Lonza) with 3% human serum (Euroclone) and 100 IU/ml penicillin/streptomycin. For *in vivo* experiments NALM-6 cell line was transduced with a lentiviral vector encoding for the secreted luciferase Lucia (Lucia+/NGFR+/NALM-6), as previously reported.³⁰

### Multi-parametric flow cytometry

HuSGM3 peripheral blood samples were obtained at day 14 after CAR T-cell infusion and stained with monoclonal antibodies specific for human CD3 BV605 (clone SK7), CD8 BV650 (clone SK1), CD4 (L3T4) BUV496 (clone SK3), CD57 BB515 (clone NK-1), CD223 (LAG-3) APC-R700 (clone T47-530), CD45RA APC-H7 (clone HI100), TIGIT BV421 (clone 741182), CD279 (PD-1) BV480 (clone EH12.1), CD27 BV750 (clone L128), CD25 (IL-2 Receptor α chain) BUV563 (clone 2A3), CD62L (L-selectin) BUV805 (clone DREG-56), CD95 (Fas/APO-1) PE-Cy™7 (clone DX2), CD28 PE-Cy™5 (clone CD28.2), CD45 APC (clone HI30), CD272 (BTLA) BB700 (clone J168-540), CD197 (CCR7) PE (clone 150503), CD271 (NGF Receptor) BUV395 (clone C40-1457), CD98 BUV661 (clone UM7F8), CD154 BUV737 (clone TRAP1) (BD Biosciences). Samples were stained in brilliant staining buffer (BD). In addition, CAR T-cell and mouse samples were stained with one or more of the following conjugated monoclonal antibodies: CD3 PB (Biolegend, cloneHIT3a), CD45 BV510 (Biolegend, clone HI30), CD271 PE-Cy7 (Biolegend, clone CD40-1457), CD271 PE (BD, clone C40-1457), CD4 FITC (Biolegend, clone SK3), anti-mouse CD45 PerCP (Biolegend, clone 30-f11), CD14 APC (Biolegend, clone M5E2), CD19 APC/Cy7 (Biolegend, clone HIB19), HLA-DR APC/Cy7 (Biolegend, clone L243), CD45RA FITC (Biolegend, clone HI100), CD62L APC (Biolegend, clone DREG-56), CD8 PerCP (BD, clone SKI). Flow-cytometry data were acquired using BD Symphony and BD Canto II cell analyzers and visualized with FlowJo_V10 software.

### EXAMPLES

### CAR T_{BULK} display a more pronounced effector signature compared to CAR T_{N/SCM} in vitro

With the aim of uncovering if pre-selection of early memory subsets as starting sources for manufacturing could enhance the therapeutic potential of CAR T cells, the inventors FACS-sorted CD62L+/CD45RA+ T_{N/SCM} cells with a purity of ∼99,1% and employed bulk unselected T cells for comparison. Both T_{N/SCM} and T_{BULK} were activated with the TransAct nanomatrix, transduced to express a CD28 co-stimulated CD19 CAR and expanded with IL-7 and IL-15 (Figure 1A).

Quite surprisingly, phenotypical characterization at the end of culture pointed out no differences in the enrichment of T-cell memory subsets between CAR T_{N/SCM} and CAR T_{BULK} and revealed a similar CD4/CD8 ratio (Figure 1B). However, a lower activation profile in terms of HLA-DR expression and a reduced expansion was characteristic of CAR T_{N/SCM}, as compared to CAR T_{BULK} (Figure 1C, 1D).

To evaluate if the two CAR T-cell products exhibited different functional capabilities, the inventors challenged them against multiple CD19+ leukemia cell lines. As expected, CAR T_{N/SCM} displayed a reduced de-granulation capability (Figure 6A), a lower cytotoxic potential (Figure 1E) and a decreased production of pro-inflammatory cytokines, as compared to CAR T_{BULK} (Figure 1F). In contrast, a similar proliferation response was detected between CAR T_{N/SCM} and CAR T_{BULK} (Figure 1G).

These data indicate that, despite phenotypical similarities, the two CAR T-cell products are functionally different, with CAR T_{BULK} showing a more pronounced effector signature compared to CAR T_{N/SCM}.

### CAR T_{N/SCM} underperform CAR T_{BULK} against aggressive leukemia in NSG mice

With the aim of assessing the anti-tumor activity of CAR T_{N/SCM} and CAR T_{BULK} *in vivo,* the inventors challenged low-dose CAR T cells in xenograft mouse models against either an indolent (i.e. ALL-CM) or an aggressive (i.e. Lucia+/NGFR+/NALM-6) B-cell leukemia (Figure 2A).

In the indolent model, even though CAR T_{N/SCM} expanded more than CAR T_{BULK} (Figure 2B), both CAR T-cell populations controlled leukemia growth in the peripheral blood (Figure 2C) and in the bone marrow (Figure 2D), leading to super-imposable survival curves in all treated mice (Figure 2E).

Differently, in the aggressive tumor model, CAR T_{BULK} significantly outperformed CAR T_{N/SCM}, both in terms of CAR T-cell expansion (Figure 2F) and tumor eradication (Figure 2G, H), while cytokine production was similar (Figure 2I). Importantly, the superiority of CAR T_{BULK} over CAR T_{N/SCM} was confirmed when NALM-6-bearing mice were treated with higher CAR T-cell doses. Indeed, although leukemia relapse experienced by CAR T_{N/SCM} was delayed when compared to the previous experiment, mice treated with CAR T_{N/SCM} eventually relapsed, failing to control leukemia growth as opposed to CAR T_{BULK} (Figure 7A). Moreover, lower tumor accumulation in the bone marrow, spleen and liver was observed in mice treated with CAR T_{BULK} at sacrifice, together with a higher CAR T-cell enrichment (Figure 7B).

While the brisk and potent antitumor activity driven by CAR T_{BULK} was in line with the greater effector functions observed *in vitro,* failure of CAR T_{N/SCM} to mount an efficient and long-lasting antitumor response was largely unexpected, leading us to reconsider the suitability of the NSG mouse model for fully addressing CAR T-cell fitness issues.

### CAR T_{N/SCM} are uniquely able to elicit recall anti-tumor responses in HSPC-humanized mice

The inventors reasoned that reduced *in vivo* efficacy by CAR T_{N/SCM} in NSG mice could be dependent on either tumor aggressiveness or intrinsic CAR T_{N/SCM} dependence on supportive human cells and cytokines, which are absent in classical xenograft mouse models. To address this issue, the inventors sought to employ the Hematopoietic Stem/Precursor Cell (HSPC)-humanized mouse model in triple transgenic SGM3 mice, which better support human healthy and tumor hematopoiesis compared to standard NSG.^{29,31} In this model, the inventors previously reported that the presence of human myeloid cells is crucial to trigger CRS and neurotoxicity.²⁹ The inventors here hypothesized that this complex human network, which includes human hematopoietic cells and cytokines, could also be instrumental to appreciate the full antitumor potential of CAR T_{N/SCM}.

The inventors therefore reconstituted SGM3 mice with human cord blood CD34+ cells and infused humanized mice (HuSGM3) with NALM-6 leukemia. Leukemia-bearing mice were then treated with high doses of CAR T_{N/SCM} or CAR T_{BULK} and monitored for T-cell expansion, tumor progression and overt toxicities. Differently to what observed in NSG mice, leukemia control was equally achieved by both CAR T_{N/SCM} and CAR T_{BULK} in HuSGM3 mice, even though CAR T-cell expansion was higher when looking at CAR T_{N/SCM} treated mice (Figure 8A and 8B). These results support the hypothesis that CAR T_{N/SCM} are more dependent on supportive homeostatic cytokines for exerting their full potential. Notably, in this experimental setting, characterized by a low tumor burden, mice did not experience severe CRS (sCRS), as indicated by only moderate and reversible weight loss and modest elevation of serum levels of IL-6 and Amyloid A (SAA), a murine homolog to the human CRS biomarker C-reactive protein²⁹ (Figure 8C).

To further challenge the therapeutic potential of the two CAR T-cell populations, the inventors performed a similar experiment in HuSGM3 mice, where the inventors injected a lower T-cell dose and provided a second tumor re-challenge (Figure 3A). In this setting, CAR T_{N/SCM} showed comparable activity to CAR T_{BULK} during the first antitumor response but were uniquely able to elicit recall responses upon leukemia re-challenge (Figure 3B). This improved therapeutic potential was associated with a higher CAR T-cell expansion, which was even more evident during the second response (Figure 3C). Accordingly, a trend towards higher IFN-γ production by CAR T_{N/SCM} was observed during the first and second antitumor responses (Figure 3D). Notably, 14 days after infusion, CAR T_{N/SCM} comprised an increased percentage of T_{CM} compared to CAR T_{BULK} (Figure 3E), possibly accounting for their superior and long-lasting therapeutic activity. Also in this setting, no signs of sCRS were detected, independently of the CAR T-cell population employed, as indicated by absence of weight loss and only moderate elevation of serum IL-6 and SAA (Figure 3F, G).

Collectively, these results indicate that HuSGM3 mice offer the appropriate human environment to support the activity of CAR T_{N/SCM}, which strongly outperformed CAR T_{BULK} in terms of long-term therapeutic potential.

### BH-SNE algorithm identifies a best performing phenotype typical of CAR T_{N/SCM}

The selective enrichment of T_{CM} in mice treated with CAR T_{N/SCM} as compared to CAR T_{BULK} prompted us to investigate whether the functional differences between the two populations could be reflected in a different phenotype once *in vivo.* To answer this question, the inventors performed the same experiment as described in Figure 3A, but with the aim of deepening the phenotypic characterization of CAR T cells after the first leukemia encounter, i.e. at day 14 after CAR T-cell infusion. To this aim, the inventors sought to employ an unsupervised approach based on the BH-SNE dimensionality reduction algorithm for data analysis.^{32,33}

As formerly observed, no difference in the capability of controlling leukemia growth was observed between CAR T_{N/SCM} and CAR T_{BULK} (Figure 9A). However, unsupervised and stochastic data downscaling, in which ∼74000 CD3+ lymphocytes were chosen for each file, together with the multi-dimensionality reduction operated by BH-SNE analysis, revealed the enrichment of clusters in totally distinct areas between CAR T_{N/SCM} and CAR T_{BULK} (Figure 4A, B). Examination of these clusters, in which an equal distribution of each sample was found (Figure 9B), highlighted intrinsic differences in the phenotypic composition of CAR T_{N/SCM} when compared to CAR T_{BULK}. Of notice, clusters of CAR T_{N/SCM} were extremely enriched in T_{SCM} and T_{CM}, whereas those concerning CAR T_{BULK} preferentially exhibited an effector memory and effector memory RA+ phenotype (Figure 4C). Moreover, CAR T_{N/SCM} displayed an activated phenotype, characterized by co-expression of activation markers and limited enrichment of inhibitory receptors, while CAR T_{BULK} were typified by an exhausted phenotype, co-expressing multiple inhibitory receptors in the absence of activation markers (Figure 4D). Indeed, the opposed spatial orientation of CAR T_{N/SCM} and CAR T_{BULK} was directed towards the enrichment of either activation or inhibitory receptors, respectively, as evidenced by the heat-map visualization (Figure 4E).

In conclusion, this unsupervised approach revealed that CAR T_{N/SCM} are endowed with enhanced *in vivo* fitness, which relies on an improved preservation of early memory cells, higher activation and lower exhaustion.

### CAR T_{N/SCM} display a negligible intrinsic potential to cause sCRS and neurotoxicity

Concerned about the higher expansion rate displayed by CAR T_{N/SCM}, which may theoretically increase their toxic potential, the inventors modified the previous experimental setting in HuSGM3 mice to exacerbate their intrinsic potential to elicit sCRS and neurotoxicity. Since such adverse events are known to be associated with both tumor burden and the level of CAR T-cell expansion upon infusion ^{11,34,35}, the inventors increased leukemia load and CAR T-cell dose of about one Log. In these conditions, CAR T_{N/SCM} and CAR T_{BULK} were mutually able to control leukemia growth, even though CAR T_{N/SCM} showed a slightly slower kinetic of tumor clearance (Figure 5A). Despite similar antitumor activity, CAR T_{N/SCM} proliferated more than CAR T_{BULK}, confirming that these cells are endowed with a superior expansion potential *in vivo* (Figure 5B). Strikingly, however, more than 50% of mice receiving CAR T_{BULK} experienced a remarkably increased sCRS, characterized by drastic weight loss, culminating in the death of the treated mice, in contrast to CAR T_{N/SCM} counterpart, in which mice weight was eventually recovered (Figure 5C, D). According to what observed in patients and in previous preclinical studies,^{11,16,29,36} the development of sCRS in mice treated with CAR T cells was associated with higher serum levels of IL-6 and SAA, both increased in the CAR T_{BULK} compared to CAR T_{N/SCM} treated group (Figure 5E,F). Besides IL-6, a wide plethora of pro-inflammatory cytokines, released by immune components in concert with activated CAR T cells, was measured in all treated mice but, once again, overall cytokine levels were lower in mice receiving CAR T_{N/SCM} than in those injected with CAR T_{BULK} (Figure 5G). Heat-map visualization of cytokine levels and composition confirmed this picture and revealed greater amounts of myeloid-derived cytokines, including IP-10, IL-8 and MCP-1, in the CAR T_{BULK} treated mice compared to CAR T_{N/SCM} (Figure 5H).

Finally, with the aim of evaluating sings of possible neurotoxic events concomitant to sCRS development, mouse brains were collected at sacrifice and subjected to histopathological evaluation. Impressively, 3 out of 6 CAR T_{BULK} treated mice showed multifocal hemorrhages,³⁷ whereas, in the group treated with CAR T_{N/SCM} only one mouse presented a small hemorrhagic focus. Of note, an increased, although apparently harmless infiltration of CD3+ cells, in the absence of CD19+ cells (Figure 10A), was found in the meninges of CAR T_{N/SCM} treated animals, as opposed to CAR T_{BULK}, in which cellular infiltration was barely detectable (Figure 5I, Table5).

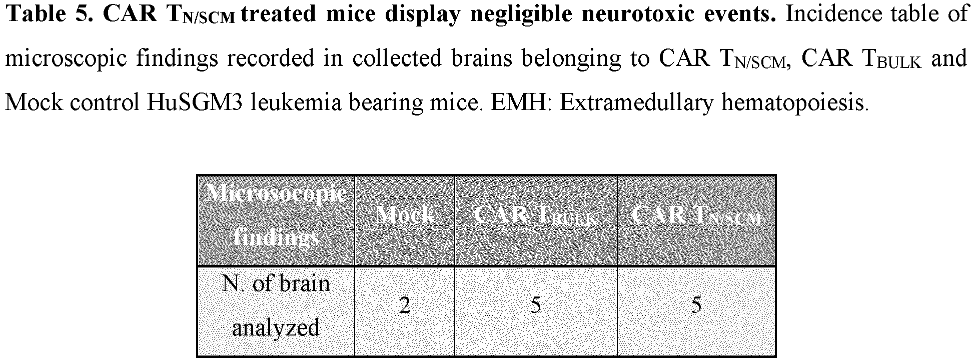

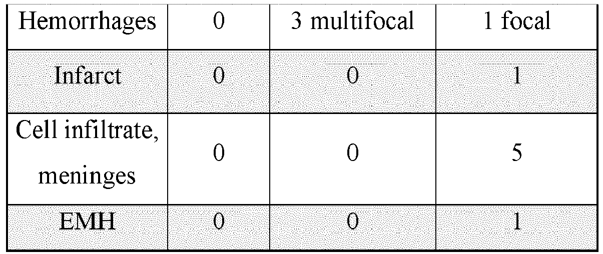

Taken together, these results indicate that, despite a greater expansion potential, CAR T_{N/SCM} are intrinsically less prone than CAR T_{BULK} to trigger detrimental CAR T cell-related toxicities, displaying a better balance between efficacy and safety profiles.

CAR T-cell fitness and antitumor activity can be enhanced through the enrichment of early memory subsets in the final cell product, by exploiting optimized manufacturing protocols.^{12,17,23} However, whether pre-selecting specific T-cell populations before manipulation would be really beneficial is still an open issue, due to the paucity of comprehensive *in vivo* data and lack of toxicity profiling. Moreover, so far, the majority of studies have compared memory T-cell subsets with each other and not with total T lymphocytes, which are the principal cell source employed in clinical trials. Even when bulk T cells were considered as reference, stimulation with suboptimal manufacturing protocols was employed.^{17,27,38} In this work, the inventors adapted the HSPC-humanized mouse model the inventors recently developed²⁹ to investigate the efficacy and safety profiles of CAR T cells generated from pre-selected T_{N/SCM} or total T lymphocytes employing a gold-standard procedure, based on stimulation with αCD3/CD28 nanomatrix and culture with IL-7/IL-15. Increasing body of evidence suggests that CAR endo-costimulation dramatically influence CAR T-cell fitness, with CD28 imprinting a prominent effector signature and 4-1BB inducing enhanced persistence and reduced differentiation.^{4,39,40} The inventors therefore reasoned that conceiving T_{N/SCM} with the typical CD28-effector capabilities could deliver the right balance to increase long-term persistence, without threatening efficient and rapid tumor de-bulking. Accordingly, while being less potent *in vitro,* CAR T_{N/SCM} mediated strong and durable antitumor responses in HSPC-humanized mice. Improved activity was accompanied by high T-cell expansion rates, which allowed unbalancing the Effector:Target ratio in favor of T cells. Of notice, highly proliferating CAR T_{N/SCM} maintained a relevant pool of early memory T cells after the first response and were less exhausted and more activated, possibly accounting for their enhanced ability to counteract tumor re-challenge.

High CAR T-cell expansion has been associated with increased incidence and severity of CRS and neurotoxicity in patients.^{10,14-16} Unexpectedly, however, CAR T_{N/SCM} failed to induce sCRS in HSPC-humanized mice and featured lower systemic cytokine levels, compared to CAR T_{BULK}. A clinical correlate to this finding is the observation that the employment of unselected CD8+ T cells compared to sorted T_{CM} CD8+ cells for CAR T-cell manufacturing was associated with an increased risk of developing sCRS.^{14,18} Importantly, the inventors also observed that mice receiving CAR T_{BULK} and experiencing sCRS showed multifocal brain hemorrhages, which were absent in mice treated with CAR T_{N/SCM}. Being similar to the events described in patients suffering from severe neurotoxicity in clinical trials, the inventors interpreted these manifestations as clear signs of neurotoxicity, resulting from endothelial damage.^{15,16} Interestingly, CAR T-cell infiltration of the brain was only detected in the CAR T_{N/SCM} group, but associated with negligible vascular disruption, supporting the notion that CAR T-cell presence *per se* is not sufficient to trigger neurotoxic events, as previously reported.^{13,41} Interestingly, while CRS and neurotoxicity induction by CAR T_{BULK} was dependent on the tumor burden and T-cell dose, CAR T_{N/SCM} proved to be intrinsically safer, offering a unique option to limit patients' risk of developing fatal toxicities, while increasing efficacy.
Toxic manifestations and antitumor activity are the result of complex pleiotropic and contact-dependent interactions taking place between activated CAR T cells and innate immune cells.^{29,36} Considering the slower CAR T_{N/SCM} kinetic of action, the inventors hypothesized that their inferior yet progressive activation was capable of stimulating innate immune cells at sufficient levels for mediating supportive antitumor activity, without triggering detrimental side effects. Recent data suggest that diminishing signal strength in CAR T cells can results in lower toxicity and enhanced antitumor activity.⁴²⁻⁴⁴ Based on their indolent functionality, the inventors hypothesized that CAR T_{N/SCM} are capable of differently processing the signal strength delivered by the CAR molecule *per se,* thus resulting in improved efficacy and safety profiles.
Strikingly, the superiority of CAR T_{N/SCM} over CAR T_{BULK} was not evident in standard xenograft mouse models, suggesting that this T-cell population could suffer from a more prominent dependence on external factors, such as supportive homeostatic cytokines. For this reason, the inventors sought to exploit the HSPC-humanized mouse model,²⁹ where the presence of innate immune cells and cytokines offers a unique human network to uncover the full antitumor potential of CAR T_{N/SCM}. In keeping with this, the inventors reasoned that the conflicting results obtained in other studies exploiting standard NSG mouse models^{17,27,38} could be reflected by the administration of soluble IL-15,^{17,27} as well as by differences in tumor types,³⁸ control T-cell populations and manufacturing procedures.^{17,27,38}
It has been reported that the frequency of T_{N/SCM} in heavily-pretreated cancer patients can be extremely variable.^{12,45-48} However, the pre-selection step could be highly beneficial to get rid of dysfunctional T cells, increasing CAR T-cell quality and lowering the dose required to achieve antitumor efficacy.²⁸ Moreover, the superiority of CAR T_{N/SCM} could be successfully exploited in the allogeneic setting, thus overcoming patient-intrinsic T-cell defects and ensuring a widespread accessibility to therapy.⁴⁹ In both scenarios, pre-selection of T_{N/SCM} could allow reducing patient-to-patient variability and better comparing the results among different clinical trials.
Taken together, our results clearly indicate that pre-selection of T_{N/SCM} can lead to a better balance between T-cell efficacy and safety profiles, significantly improving the therapeutic index of current T-cell therapies in particular CAR T-cell therapies.

### References

1. Maude SL, Laetsch TW, Buechner J, et al. Tisagenlecleucel in children and young adults with B-cell lymphoblastic leukemia. N. Engl. J. Med. 2018;
2. Park JH, Rivière I, Gonen M, et al. Long-term follow-up of CD19 CAR therapy in acute lymphoblastic leukemia. N. Engl. J. Med. 2018;
3. Porter DL, Hwang WT, Frey N V., et al. Chimeric antigen receptor T cells persist and induce sustained remissions in relapsed refractory chronic lymphocytic leukemia. Sci. Transl. Med. 2015;
4. Majzner RG, Mackall CL. Clinical lessons learned from the first leg of the CAR T cell journey. Nat. Med. 2019;
5. Seimetz D, Heller K, Richter J. Approval of First CAR-Ts: Have we Solved all Hurdles for ATMPs? Cell Med. 2019;
6. Turtle CJ, Hay KA, Hanafi LA, et al. Durable molecular remissions in chronic lymphocytic leukemia treated with CD19-Specific chimeric antigen Receptor-modified T cells after failure of ibrutinib. J. Clin. Oncol. 2017;
7. Geyer MB, Rivière I, Sénéchal B, et al. Autologous CD19-Targeted CAR T Cells in Patients with Residual CLL following Initial Purine Analog-Based Therapy. Mol. Ther. 2018;
8. Neelapu SS, Locke FL, Bartlett NL, et al. Axicabtagene ciloleucel CAR T-cell therapy in refractory large B-Cell lymphoma. N. Engl. J. Med. 2017;
9. Lim WA, June CH. The Principles of Engineering Immune Cells to Treat Cancer. Cell. 2017;
10. Hirayama A V., Turtle CJ. Toxicities of CD19 CAR-T cell immunotherapy. Am. J. Hematol. 2019;
11. Brudno JN, Kochenderfer JN. Recent advances in CAR T-cell toxicity: Mechanisms, manifestations and management. Blood Rev. 2019;
12. Gattinoni L, Speiser DE, Lichterfeld M, Bonini C. T memory stem cells in health and disease. Nat. Med. 2017;
13. Maude SL, Frey N, Shaw PA, et al. Chimeric antigen receptor T cells for sustained remissions in leukemia. N. Engl. J. Med. 2014;
14. Hay KA, Gauthier J, Hirayama A V., et al. Factors associated with durable EFS in adult B-cell ALL patients achieving MRD-negative CR after CD19 CAR T-cell therapy. Blood. 2019;
15. Gust J, Hay KA, Hanafi LA, et al. Endothelial activation and blood-brain barrier disruption in neurotoxicity after adoptive immunotherapy with CD19 CAR-T cells. Cancer Discov. 2017;
16. Santomasso B, Bachier C, Westin J, Rezvani K, Shpall EJ. The Other Side of CAR T-Cell Therapy: Cytokine Release Syndrome, Neurologic Toxicity, and Financial Burden. Am. Soc. Clin. Oncol. Educ. B. 2019;
17. Sabatino M, Hu J, Sommariva M, et al. Generation of clinical-grade CD19-specific CAR-modified CD81 memory stem cells for the treatment of human B-cell malignancies. Blood. 2016;
18. Turtle CJ, Hanafi LA, Berger C, et al. CD19 CAR-T cells of defined CD4+:CD8+ composition in adult B cell ALL patients. J. Clin. Invest. 2016;
19. Flynn JK, Gorry PR. Stem memory T cells (TSCM)-their role in cancer and HIV immunotherapies. Clin. Transl. Immunol. 2014;
20. Fraietta JA, Lacey SF, Orlando EJ, et al. Determinants of response and resistance to CD19 chimeric antigen receptor (CAR) T cell therapy of chronic lymphocytic leukemia. Nat. Med. 2018;
21. Fraietta JA, Nobles CL, Sammons MA, et al. Disruption of TET2 promotes the therapeutic efficacy of CD19-targeted T cells. Nature. 2018;
22. Gattinoni L, Lugli E, Ji Y, et al. A human memory T cell subset with stem cell-like properties. Nat. Med. 2011;
23. Cieri N, Camisa B, Cocchiarella F, et al. IL-7 and IL-15 instruct the generation of human memory stem T cells from naive precursors. Blood. 2013;
24. Bondanza A, Valtolina V, Magnani Z, et al. Suicide gene therapy of graft-versus-host disease induced by central memory human T lymphocytes. Blood. 2006;
25. Mock U, Nickolay L, Philip B, et al. Automated manufacturing of chimeric antigen receptor T cells for adoptive immunotherapy using CliniMACS prodigy. Cytotherapy. 2016;
26. Kaneko S, Mastaglio S, Bondanza A, et al. IL-7 and IL-15 allow the generation of suicide gene modified alloreactive self-renewing central memory human T lymphocytes. Blood. 2009;
27. Pilipow K, Scamardella E, Puccio S, et al. Antioxidant metabolism regulates CD8+ T memory stem cell formation and antitumor immunity. JCI insight. 2018;
28. Klebanoff CA, Scott CD, Leonardi AJ, et al. Memory T cell-driven differentiation of naive cells impairs adoptive immunotherapy. J. Clin. Invest. 2016;
29. Norelli M, Camisa B, Barbiera G, et al. Monocyte-derived IL-1 and IL-6 are differentially required for cytokine-release syndrome and neurotoxicity due to CAR T cells. Nat. Med. 2018;
30. Falcone L, Casucci M. Exploiting secreted luciferases to monitor tumor progression in vivo. Methods Mol. Biol. 2016;
31. Billerbeck E, Barry WT, Mu K, et al. Development of human CD4+FoxP3+ regulatory T cells in human stem cell factor-, granulocyte-macrophage colony-stimulating factor-, and interleukin-3-expressing NOD-SCID IL2Rγnull humanized mice. Blood. 2011;
32. Noviello M, Manfredi F, Ruggiero E, et al. Bone marrow central memory and memory stem T-cell exhaustion in AML patients relapsing after HSCT. Nat. Commun. 2019;
33. Yang Z, Peltonen J, Kaski S. Scalable optimization of neighbor embedding for visualization. 30th Int. Conf. Mach. Learn. ICML 2013. 2013;
34. Maude SL, Barrett D, Teachey DT, Grupp SA. Managing cytokine release syndrome associated with novel T cell-engaging therapies. Cancer J. (United States). 2014;
35. Davila ML, Riviere I, Wang X, et al. Efficacy and toxicity management of 19-28z CAR T cell therapy in B cell acute lymphoblastic leukemia. Sci. Transl. Med. 2014;
36. Giavridis T, Van Der Stegen SJC, Eyquem J, et al. CAR T cell-induced cytokine release syndrome is mediated by macrophages and abated by IL-1 blockade letter. Nat. Med. 2018;
37. Kaufmann W, Bolon B, Bradley A, et al. Proliferative and Nonproliferative Lesions of the Rat and Mouse Central and Peripheral Nervous Systems. Toxicol. Pathol. 2012;40(4):87S-157S.
38. Sommermeyer D, Hudecek M, Kosasih PL, et al. Chimeric antigen receptor-modified T cells derived from defined CD8+ and CD4+ subsets confer superior antitumor reactivity in vivo. Leukemia. 2016;
39. Kawalekar OU, Posey AD, Fraietta J, et al. Distinct Signaling By Chimeric Antigen Receptors (CARs) Containing CD28 Signaling Domain Versus 4-1BB In Primary Human T Cells. Blood. 2013;
40. Salter AI, Ivey RG, Kennedy JJ, et al. Phosphoproteomic analysis of chimeric antigen receptor signaling reveals kinetic and quantitative differences that affect cell function. Sci. Signal. 2018;
41. Santomasso BD, Park JH, Salloum D, et al. Clinical and biological correlates of neurotoxicity associated with car t-cell therapy in patients with B-cell acute lymphoblastic leukemia. Cancer Discov. 2018;
42. Feucht J, Sun J, Eyquem J, et al. Calibration of CAR activation potential directs alternative T cell fates and therapeutic potency. Nat. Med. 2019;
43. Ying Z, Huang XF, Xiang X, et al. A safe and potent anti-CD19 CAR T cell therapy. Nat. Med. 2019;
44. Ghorashian S, Kramer AM, Onuoha S, et al. Enhanced CAR T cell expansion and prolonged persistence in pediatric patients with ALL treated with a low-affinity CD19 CAR. Nat. Med. 2019;
45. Xu L, Yao D, Tan J, et al. Memory T cells skew toward terminal differentiation in the CD8+ T cell population in patients with acute myeloid leukemia. J. Hematol. Oncol. 2018;
46. Vahidi Y, Faghih Z, Talei AR, Doroudchi M, Ghaderi A. Memory CD4+ T cell subsets in tumor draining lymph nodes of breast cancer patients: A focus on T stem cell memory cells. Cell. Oncol. 2018;
47. Singh N, Perazzelli J, Grupp SA, Barrett DM. Early memory phenotypes drive T cell proliferation in patients with pediatric malignancies. Sci. Transl. Med. 2016;
48. Das RK, Vernau L, Grupp SA, Barrett DM. Naive T-cell deficits at diagnosis and after chemotherapy impair cell therapy potential in pediatric cancers. Cancer Discov. 2019;
49. Depil S, Duchateau P, Grupp SA, Mufti G, Poirot L. 'Off-the-shelf' allogeneic CAR T cells: development and challenges. Nat. Rev. Drug Discov. 2020;

## Claims

1. A method to produce a T cell comprising the step of:
a) isolating a population of CD45RA⁺/CD62L⁺/CD95⁻ T cells and CD45RA⁺/CD62L⁺/CD95⁺ T cells from a biological sample of a subject;
b) activating said population of T cells by stimulating CD3 and CD28;
c) contacting said activated population of T cells with IL-7 and IL-15.

2. The method according to claim 1 wherein said T cell has at least one of the following properties: prevent cytokine release syndrome, prevent neurotoxicity, display a high expansion rate, preserved early memory phenotype, a poor exhausted profile and long-term persistence.

3. The method according to claim 1 or 2 further comprising introducing in said population of T cells a nucleic acid sequence encoding an exogenous gene, thereby producing an engineered T cell.

4. The method according to any one of claim 3 wherein the exogenous gene encodes an antigen-recognizing receptor, an ortho-receptor, an immunomodulatory cytokine, a chemokine receptor, a dominant negative receptor, a transcription factor able to prevent exhaustion, preferably the antigen is a tumor antigen, a self-antigen or a pathogen antigen.

5. The method according to claim 4 wherein said antigen recognizing receptor is a T cell receptor (TCR) or a chimeric antigen receptor (CAR).

6. The method according to claim 4 or 5 wherein said antigen recognizing receptor is exogenous.

7. The method according to any one of claim 4 to 6 wherein said nucleic acid sequence is introduced by a vector.

8. The method according to any one of previous claim wherein said nucleic acid sequence is placed at an endogenous gene locus of the T cell.

9. The method according to any one of previous claim wherein said insertion of the nucleic acid sequence disrupts or abolishes the endogenous expression of a TCR.

10. A T cell or an engineered T cell produced by the method of any one of previous claims.

11. An isolated engineered CD45RA⁺/CD62L⁺/CD95⁻ T cells and CD45RA⁺/CD62L⁺/CD95⁺ T cell population comprising a nucleic acid sequence encoding an exogenous gene wherein said population reduces at least one symptom of cytokine release syndrome (CRS) or reduces at least one symptom of neurotoxicity in a subject or wherein said population has high expansion rate.

12. The T cell or the engineered T cell according to claim 10 or the isolated engineered T cell population according to claim 11 or a pharmaceutical composition comprising the T cell or the engineered T cell according to claim 10 or the isolated engineered T cell population according to claim 11 for use in a therapy, preferably for use in reducing tumor burden or for use in treating and/or preventing a neoplasm or for use in lengthening survival of a subject having a neoplasm or for use in the treatment of an infection or for use in the treatment of an autoimmune disease, preferably the neoplasm is selected from the group consisting of solid or blood cancer, preferably B cell leukemia, multiple myeloma, acute lymphoblastic leukemia (ALL), chronic lymphocytic leukemia, acute myeloid leukemia (AML), non- Hodgkin's lymphoma, preferably the neoplasm is B cell leukemia, multiple myeloma, lymphoblastic leukemia (ALL), chronic lymphocytic leukemia, or non- Hodgkin's lymphoma.

13. The T cell or the engineered T cell according to claim 10 or the isolated engineered T cell population according to claim 11 or a pharmaceutical composition comprising the T cell or the engineered T cell according to claim 10 or the isolated engineered T cell population according to claim 11 for use for use in preventing and/or reducing at least one symptom of cytokine release syndrome (CRS) or for use in reducing at least one symptom of neurotoxicity in a subject.

14. The T cell or the engineered T cell according to claim 10 or the isolated engineered T cell population according to claim 11 or a pharmaceutical composition comprising the T cell or the engineered T cell according to claim 10 or the isolated engineered T cell population according to claim 11 for use according to claim 13 wherein the at least one symptom of cytokine release syndrome is reducing the level of at least one cytokine or chemokine or other factor selected from the group consisting of: IL-1 alpha, IL-1 beta, IL-6, IL-8, IL-10, TNF-a, IFN-g, IL-5, IL-2, IL-4, G-CSF, GM-CSF, M-CSF, TGF-b, IL-12, IL-15, and IL-17, IP-10, MIP-1-alpha, MCP1, von Willebrand factor, Angiopoietin 2, SAA, protein C reactive, ferritin.
